# EUROPEAN PATENT SPECIFICATION

(11) **EP 4 215 232 B1**
(45) Date of publication and mention of the grant of the patent: **29.01.2025**
(21) Application number: 22216961.7
(22) Date of filing: 28.12.2022
(51) Int. Cl.: A61M 11/00, A61M 16/04, A61M 16/08, A61M 16/14, A61M 16/16

(54) **A FLUID DELIVERY SYSTEM FOR DELIVERY OF AEROSOLIZED LIQUID TO A VENTILATED PATIENT**
FLÜSSIGKEITSABGABESYSTEM ZUR ABGABE EINER AEROSOLISIERTEN FLÜSSIGKEIT AN EINEN BEATMETEN PATIENTEN
SYSTÈME DE DISTRIBUTION DE FLUIDE POUR LA DISTRIBUTION D'UN LIQUIDE AÉROSOLISÉ À UN PATIENT VENTILÉ

(30) Priority: 29.12.2021 US 202163294671 P; 22.07.2022 US 202263391543 P; 13.12.2022 US 202218065056
(43) Date of publication of application: 26.07.2023
(73) Proprietor: Covidien LP, Mansfield, MA 02048 (US)
(72) Inventor: Phillips, Karen Ann, Carlsbad, 92008 (US); Phillips, Matthew J., Carlsbad, 92008 (US); Novkov, Donald J., Carlsbad, 92008 (US); Kaus, Stanley B., Boulder, 80301 (US); Sanchez, Gabriel, Carlsbad, 92008 (US); Rachlin, Thomas M., Carlsbad, 92008 (US); Shaver, Scott M., Boulder, 80301 (US); Dreher, Frank M., Boulder, 80301 (US); Honeycutt, Alexander J., Carlsbad, 92008 (US)
(74) Representative: Marks & Clerk LLP

(56) References cited:
- WO-A1-2008/056986
- WO-A1-2019/222159
- FR-A1- 2 624 745
- US-A- 4 739 756
- US-A- 4 953 547
- US-A- 5 605 147
- US-A- 5 947 120
- US-A1- 2017 035 983
- US-B1- 6 237 597

## Description

### CROSS-REFERENCE TO RELATED APPLICATIONS

This application claims the benefit of U.S. Provisional Application No. 63/294,671 filed December 29, 2021, entitled "Fluid Delivery System for Medical Ventilation," and U.S. Provisional Application No. 63/391,543 filed July 22, 2022, entitled "Nebulizer Systems and Methods".

### INTRODUCTION

Medical ventilator systems have long been used to provide ventilatory and supplemental oxygen support to patients. These ventilators typically comprise a connection for pressurized gas (air, oxygen) that is delivered to the patient through a conduit or tubing. Some ventilator systems are used with humidifiers to humidify the gas delivered to the patient and to improve patient adherence and comfort.

It is with respect to this general technical environment that aspects of the present technology disclosed herein have been contemplated. Furthermore, although a general environment is discussed, it should be understood that the examples described herein should not be limited to the general environment identified herein.

US5605147 (A) discloses a device which allows for the rapid administration of liquid medicants into the lungs of a patient via endotracheal tube without interrupting the ventilation cycles and without the risk of displacement of endotracheal tube from the patient. An endotracheal insert apparatus that consists of: ventilation adaptor apparatus, inserted between endotracheal tube and the means for ventilation of the patient; medication injection apparatus; and medication tubing, is inserted down endotracheal tube. A syringe without needle is attached to medication injection apparatus at twist lock adaptor. The liquid medicant is then forced from the syringe and travels through medication tubing and medication tubing and expelled at the distal end of endotracheal tube through ejection port. In one alternative embodiment, the addition of collapsible sheath allows for the retraction of medication tubing from endotracheal tube when medication is not being injected.

US4953547 (A) discloses an improved drug administering respiration endotracheal system which permits simultaneous multiple injection of life-saving medication into the lungs of the patient without interruption of the flow of life supporting gasses. The system includes a connector with a linear axial passageway for gasses and two separate medication injection ports adapted to receive a hypodermic needle and a medical syringe.

US5947120 (A) discloses a substantially tubular body which includes an open first end adapted for connection to a ventilator. The body further includes an open second end opposite the first end and adapted for connection to an endotracheal tube. An injection tube has a primary end and a secondary end. A pre-pierced, self-sealing injection port is disposed at the primary end of the injection tube and is adapted for insertion of the needle there-through and into the injection tube. The secondary end is connected to the body between the first end and the second end, whereby medication delivered through a needle inserted through the primary end will flow through the injection tube, into the body, then into the endotracheal tube, and will be atomized into a patient's lungs when the ventilator forces air through the endotracheal tube.

US6237597 (B1) discloses an endotracheal medication port adapter for administering a first medication and one of a second medication and life supporting gas directly into the pulmonary vasculature of a patient. The adapter includes a first medication tube including a first medication receiving end for receiving the first medication therethrough and a second end. A ring adapter includes a first tube receiving port for receiving the second end of the first medication tube, a depositing port and a second tube receiving port. The first tube receiving port receives the second end of the first medication tube, wherein the first medication tube extends through the first tube receiving port and the depositing port for dispensing medication deposited therein into the pulmonary vasculature of the patient. A flexible membrane including a recess therein is positioned to cover the second tube receiving port for receiving and forming an airtight seal with one of a second medication tube and a source of life supporting gas and a cover is positioned to seal the second tube receiving port when one of a second medication tube and a source of life supporting gas is not received by the flexible membrane.

US2017035983 (A1) discloses an apparatus for delivering an aerosolized medication to a patient on a ventilation circuit. The apparatus comprises a multi dose metered syringe preloaded with the aerosolized medication such as albuterol. The syringe comprises a Luer lock at a bottom tip, which is removably attachable to a carbon dioxide sampling port of an endotracheal tube. A plunger is adapted to be fitted inside the syringe and actuated for delivering a predefined dose of the aerosolized medication into the endotracheal tube, without disconnecting the ventilation circuit. The delivery of aerosolized medication is synchronized with oxygen inspiration for optimal delivery.

FR2624745 (A1) makes it possible to atomize medicaments to be administered by inhalation in patients with an intubation probe or a tracheotomy cannula. The device comprises a hollow piece of which one cylindrical end fits perfectly onto the connection of the intubation probe or the tracheotomy cannula and the other cylindrical end fits perfectly onto the T-piece of the respirator. The main piece also comprises a lateral orifice for introducing the valve of the vial containing the product to be atomized. A duct prolongs the natural orifice, bends at right angles at the center of the piece and extends towards the end. The valve of the vial can be blocked by a stop located at the junction of the duct and the lateral orifice. A flexible tube attached to the free end of the duct penetrates the intubation probe or the tracheotomy cannula as far as their end in the bronchial tree. The flexible tube may be removable. The piece comprises a rough concave part diametrically opposite the lateral orifice which can be firmly gripped by the index finger of the user in order to press on the valve. The device may be sterile and for single use. Its use is recommended for all medicaments administered by inhalation in patients with an intubation probe or a tracheotomy cannula.

US4739756 (A) discloses an endotracheal tube which comprises: a main flexible hollow tube member including an annular wall for forming a large central lumen for the input transport of oxygen and for the output transport of the products of respiration or of suctioning; a small lateral lumen being located in such hollow tube member annular wall for the input transport of medication to be delivered distally to the internal lining of the lungs; and an ejection ring being located at the most distal annular surface of such hollow tube member annular wall, such ejection ring including a rear input channel for receiving the medication from such small lateral lumen, and including a plurality of uniformly spaced apart front output openings or orifices for ejecting such medication as a spray distally.

WO2019222159 (A1) describes systems and methods for humidifying ventilator delivered breathing gases. These systems and methods utilize a hollow cone atomizer (e.g., a pressure swirl atomizer) and/or a heating element associated with a heating circuit and/or a heating tube. In some aspect, the systems and methods utilize received flow, temperature, and/or humidity information to determine an amount of water to add to breathing gases to reach a desired humidity of the breathing gases delivered to the patient. In further aspects, the humidification system can serve as a nebulization system for delivering nebulized medicine.

### SUMMARY OF THE INVENTION

The present invention provides a fluid delivery system for delivery of aerosolized liquid to a ventilated patient as defined in claim 1. Further preferred embodiments of the invention are defined in the dependent claims.

### SUMMARY OF THE DISCLOSURE

Reference will now be made to various exemplary embodiments or aspects of the disclosure. Embodiments or aspects disclosed herein which do not fall under the scope of the appended claims do not form part of the present invention, but are useful for understanding the principles of the invention. The scope of the present invention is defined by the appended claims.

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

Among other things, aspects of the present disclosure include systems and methods for medication delivery for a ventilation system through a nozzle. Additional aspects of the disclosure not presently claimed include systems and methods for dual nozzle humidification.

In an embodiment, the technology relates to a fluid delivery system for delivery of aerosolized liquid to a ventilated patient. The fluid delivery system includes an endotracheal tube configured to deliver breathing gases to a patient; a multi-port connector coupled to the endotracheal tube, the multi-port connector including: a primary channel directing a flow of the breathing gases into the endotracheal tube; and a secondary channel having a first end and a second end, wherein the secondary channel is fluidly coupled to the primary channel at the second end at an angle that is 90 degrees or less from the flow of the breathing gases. The fluid delivery system also includes an injection tip configured to engage with the first end of the secondary channel and a pressure applicator, wherein the injection tip includes a set of orifices configured to aerosolize a liquid when the liquid is applied at a pressure by the pressure applicator.

In other embodiments, the pressure applicator may be one of: a syringe or a pump. In another embodiment, the set of orifices may be sized based on a viscosity of the liquid to be injected. In yet another example, the set of orifices are sized based on the pressure to be applied by the pressure applicator. In still another example, the set of orifices includes at least six orifices. In a further example, the set of orifices are distributed symmetrically about a cross section of the injection tip.

In another example, a first orifice of the set of orifices is sized differently than a second orifice of the set of orifices. In yet another example, the injection tip has a body that is substantially frustoconical. In still another example, the injection tip is removable from the first end of the secondary channel. In yet another example, the pressure applicator is a pump; and the system further includes a controller configured to cause pressurized liquid from the pump to be injected through the set of orifices during an inhalation phase of delivered ventilation, but not during an exhalation phase of the delivered ventilation.

In another aspect, the technology relates to a dual nozzle ventilation system that includes a humidification system and a fluid delivery system. The humidification system includes a first pump configured to pressurize water from a water reservoir; a first nozzle configured to atomize the water, wherein the first nozzle is positioned in a heated portion of a ventilation tubing configured to deliver breathing gases to a ventilated patient such that atomized water interacts with the heated portion of the ventilation tubing to humidify the breathing gases; a first valve to control the flow of pressurized water from the first pump into the first nozzle; and a first controller configured to control the first valve based on a flowrate of the breathing gases. The fluid delivery system includes a second pump configured to pressurize a liquid from a fluid reservoir; and a second nozzle positioned downstream in the line from the first nozzle of the humidification system, wherein the second nozzle is configured to aerosolize the liquid in the humidified breathing gases; a second valve to control the flow of pressurized liquid from the second pump into the second nozzle; and a second controller configured to control the second valve based on the flowrate of the breathing gases.

In other embodiments, the ventilation tubing may include a wye and the first nozzle and the second nozzle may be positioned downstream of the wye. In other embodiments, the ventilation tubing may include a wye and the first nozzle may be positioned upstream of the wye and the second nozzle may be positioned downstream of the wye. In a further embodiment, the second nozzle may be coupled to a multi-port connector coupled to an endotracheal tube. In still another embodiment, the humidified breathing gases may be configured to flow into a primary port of the multi-port connector; and the second nozzle may be coupled to a secondary port of the multi-port connector.

In another aspect, the technology relates to a method for delivering liquid into an invasive interface. The method includes delivering breathing gases into an invasive interface through a primary port and a primary channel of a connector, wherein the primary channel of the connector is fluidly coupled to a secondary channel of the connector; based on properties of an injection tip, calculating a cumulative duration for delivery of set volume of a liquid; pressurizing the liquid; and injecting the pressurized liquid, for the cumulative duration, into the injection tip to aerosolize the liquid, wherein the injection tip is coupled to a secondary port of the connector such that the aerosolized liquid travels through the secondary channel and is carried into the invasive interface by the breathing gases in the primary channel.

In an example, the method further includes determining a breathing phase, wherein the breathing phase is one of: an inhalation phase or an exhalation phase; and wherein injecting the pressurized liquid is performed during the inhalation phase. In another example, injecting the pressurized liquid is performed during a first half of the inhalation phase. In still another example, when the cumulative duration exceeds a duration of the inhalation phase, the pressurized liquid is delivered during multiple inhalation phases spanning at least two breaths. In still another example, the properties of the injection tip include at least one of: an inner diameter of the injection tip; a quantity of orifices on a membrane of the injection tip; or a size of the orifices.

In another aspect, the technology relates to a method for injecting liquid into a flow of breathing gases. The method includes delivering a liquid along a first fluid line from a fluid reservoir to a first nozzle and into a flow of breathing gases via a pump, wherein the pump is outside a wetted path of the liquid; removing the first fluid line and the first nozzle, wherein removing the first fluid line includes removing the first fluid line from the pump; replacing the first fluid line with a second fluid line and the first nozzle with a second nozzle, wherein the second fluid line is inserted into the pump; and delivering the liquid along the second fluid line from the fluid reservoir to the second nozzle and into the flow of breathing gases via the pump.

In an example, the first nozzle and the second nozzle are positioned to inject the liquid at an endotracheal tube. In another example, delivering the liquid along a first fluid line from a fluid reservoir to a first nozzle includes pumping the liquid into a pressure chamber. In still another example, the method further includes controlling the pump based on a pressure inside the pressure chamber. In yet another example, the method further includes controlling a valve positioned between the pressure chamber and the first nozzle based on a pressure inside the pressure chamber. In a further example, the valve is positioned outside the wetted path of the liquid. In still yet another example, the pressure chamber is a first pressure chamber, and the method further includes removing the first pressure chamber; and replacing the first pressure chamber with a second pressure chamber, wherein delivering the liquid along the second fluid line further includes delivering the liquid from the fluid reservoir to the second pressure chamber to the second nozzle.

In a further aspect, a fluid delivery system for delivery of aerosolized liquid to a ventilated patient is disclosed. The fluid delivery system includes an endotracheal tube configured to deliver breathing gases to a patient. The endotracheal tube includes an outlet end positionable inside the patient; a wall defined by an outer diameter and an inner diameter; and at least one lumen at least partially contained in the wall and having a lumen outlet at the outlet end of the endotracheal tube. The fluid delivery system also includes an injection tip having a set of orifices that, when receiving a pressurized liquid, aerosolize the liquid, wherein the injection tip is positionable proximate the outlet end of the endotracheal tube either in the at least one lumen within the wall or inside of the inner diameter.

In an example, the set of orifices are sized based on a viscosity of the liquid to be injected. In another example, the injection tip is guidable via a stylet. In a further example, the injection tip is positionable in the at least one lumen of the endotracheal tube proximate the lumen outlet. In yet another example, the at least one lumen is a first lumen, and wherein the endotracheal tube further includes a second lumen contained in the wall of the endotracheal tube, wherein a gas is directable through the second lumen to bias a flow of aerosolized liquid from the injection tip.

It is to be understood that both the foregoing general description and the following Detailed Description are explanatory and are intended to provide further aspects and examples of the disclosure.

### BRIEF DESCRIPTION OF THE DRAWINGS

The following drawing figures, which form a part of this application, are illustrative of aspects of systems and methods described below and are not meant to limit the scope of the disclosure in any manner.
FIG. 1 shows a diagram illustrating an example of a medical ventilator connected to a human patient.
FIG. 2 shows a block-diagram illustrating an example of a ventilator system.
FIGS. 3A-3D show different views of a connector for coupling ventilation tubing to an endotracheal tube.
FIG. 4 shows a fluid delivery system.
FIG. 5 shows an injection tip.
FIGS. 6A-6C show different configurations for a set of orifices of an injection tip.
FIG. 7 shows a system for delivering aerosolized fluid.
FIG. 8 shows a ventilation system including a fluid delivery system.
FIGS. 9A-9D show different configurations for a ventilation system including a fluid delivery system and a humidification system.
FIGS. 10A-10C shows components of an example fluid delivery system.
FIGS. 11A-11B show components of another example fluid delivery system.
FIG. 12 shows a flowchart illustrating a method for delivering a fluid into an invasive patient interface.
FIG. 13 shows a flowchart illustrating a method for fluid delivery.
FIGS. 14A-14B show another configuration for sets of orifices of an injection tip.
FIGS. 15A-15C show another configuration for sets of orifices of an injection tip.
FIGS. 16A-16C show another injection tip.
FIGS. 17A-17B show another injection tip.
FIGS. 18A-18E show different configurations of an injection tip and a connector for coupling ventilation tubing to an endotracheal tube.
FIGS. 19A-19D show different configurations of a connector for coupling ventilation tubing to an endotracheal tube.
FIGS. 20A-20B show a ventilation system including a fluid delivery system not presently claimed.
FIGS. 21A-21B show different configurations of a wye component of a ventilation system.
FIGS. 22A-22C show an endotracheal tube with an integrated injection tip.

While examples of the disclosure are amenable to various modifications and alternative forms, specific aspects have been shown by way of example in the drawings and are described in detail below. The intention is not to limit the scope of the disclosure to the particular aspects described. On the contrary, the disclosure is intended to cover all modifications, equivalents, and alternatives falling within the scope of the disclosure.

### DETAILED DESCRIPTION

As discussed briefly above, medical ventilator systems have long been used to provide ventilatory and supplemental oxygen support to patients. These ventilators typically comprise a connection for pressurized gas (air, oxygen) that is delivered to the patient through a conduit or tubing. Some ventilator systems are used with humidifiers to humidify the gas delivered to the patient and to improve patient adherence and comfort.

Delivery of fluids, which may be a liquid such as water or medicine, into a breathing circuit poses several challenges. For instance, when considering delivery of water to humidify gases in a breathing circuit, it is desirable to evaporate water to increase the humidity of the breathing gases at a specified temperature. Oversaturation or over-humidification, however, may cause rainout of the water in either the breathing circuit, ventilator exhalation port, and/or lungs of a patient, which can lead to infection of the patient or contamination of the breathing circuit and/or components of the ventilator. Alternatively, when considering delivery of a fluid other than water (e.g., medicine or drug), it is desirable to aerosolize the fluid and suspend droplets of the fluid in the breathing gases rather than evaporate the fluid. Additionally, delivery of a fluid to a patient may be associated with a prescribed dosage. Delivery of the fluid into the breathing circuit, however, may not result in all of the fluid reaching the lungs of the patient. For example, the fluid may stick to the interior walls of the breathing circuit or flow past the wye and be collected by an expiratory filter. Ultimately, the dispersion of the fluid may result in an improper and/or unknown dosage. Moreover, humidity may impact aerosolization or suspension of a fluid in the gases flowing through a breathing circuit.

Among other things, the systems and methods disclosed herein address these circumstances by providing a delivery system for a fluid (e.g., medicine or drug) into a breathing circuit. Additionally, the present technology provides dual nozzles for delivering and implementing humidification and medicinal dosing in the same breathing circuit. The fluid delivery systems allow a fluid to be aerosolized proximate a patient interface, such as an endotracheal tube using a connector, an injection tip, and a pressure applicator. The fluid may be aerosolized phasically with an inhalation phase of a patient and/or injections of water to increase humidification. Both a humidification system and fluid delivery system may be concurrently coupled to a breathing circuit, with two separate nozzles, and may be positioned about various portions of the breathing circuit. Aspects of a humidification system are described in U.S. Application No. 17/465,517, titled "Systems and Methods for Active Humidification in Ventilatory Support" and filed on September 2, 2021. With these concepts in mind, several examples of fluid delivery methods and systems are discussed below.

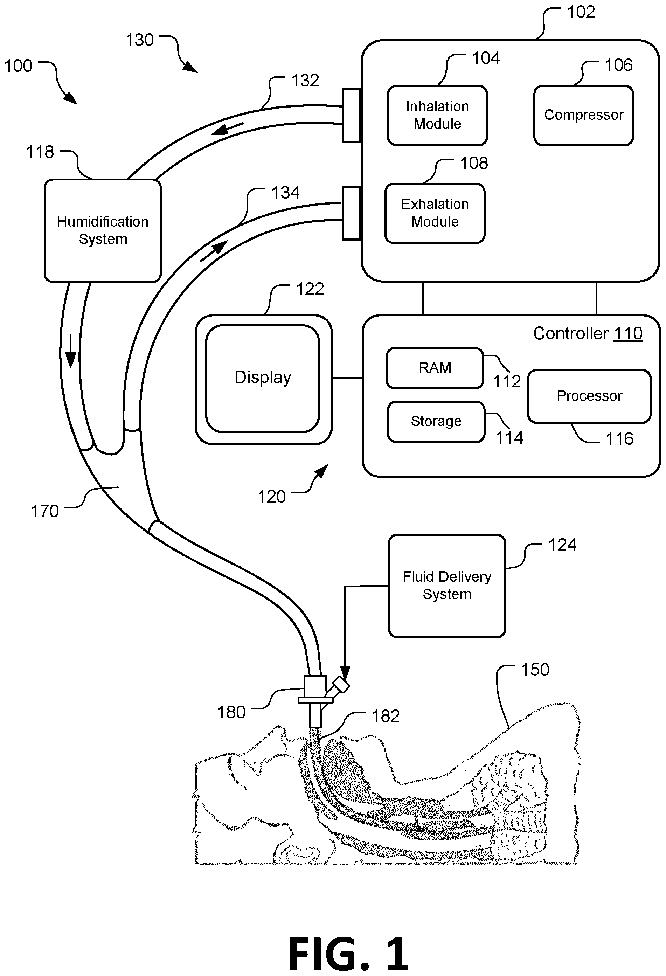

FIG. 1 is a diagram illustrating an example of a medical ventilator 100 connected to a human patient 150. The ventilator 100 may provide positive pressure ventilation to the patient 150. Ventilator 100 includes a pneumatic system 102 (also referred to as a pressure generating system 102) for circulating breathing gases to and from patient 150 via the ventilation tubing system 130, which couples the patient to the pneumatic system via a patient interface 180, such as an invasive (e.g., endotracheal tube 182, as shown) or a non-invasive (e.g., nasal mask) patient interface.

Ventilation tubing system 130 may be a two-limb (shown) or a one-limb circuit for carrying gases to and from the patient 150. In a two-limb example, a fitting, typically referred to as a "wye-fitting" 170 or wye 170, may be provided to couple a patient interface 180 to an inhalation limb 132 and an exhalation limb 134 of the ventilation tubing system 130. In the example shown in FIG. 1, the patient interface 180 is an endotracheal tube 182 coupled to the ventilation tubing with a connector. The depicted patient interface 180 (e.g., endotracheal tube 182 and connector) are used for invasive ventilation of a patient 150. Other patient interfaces may be implemented, such as non-invasive interfaces, including a nasal cannula, a mask, a nasal mask, etc.

Pneumatic system 102 may have a variety of configurations. In the present example, system 102 includes an exhalation module 108 coupled with the exhalation limb 134 and an inhalation module 104 coupled with the inhalation limb 132. Compressor 106 or other source(s) of pressurized gases (e.g., air, oxygen, and/or helium) is coupled with inhalation module 104 to provide a gas source for ventilatory support via inhalation limb 132. The pneumatic system 102 may include a variety of other components, including mixing modules, valves, sensors, tubing, accumulators, filters, etc., which may be internal or external sensors to the ventilator (and may be communicatively coupled, or capable communicating, with the ventilator).

Controller 110 is operatively coupled with pneumatic system 102, signal measurement and acquisition systems, and an operator interface 120 that may enable an operator to interact with the ventilator 100 (e.g., change ventilation settings, select operational modes, view monitored parameters, etc.). Controller 110 may include memory 112, one or more processors 116, storage 114, and/or other components of the type found in command and control computing devices. In the depicted example, operator interface 120 includes a display 122 that may be touch-sensitive and/or voice-activated, enabling the display 122 to serve both as an input and output device.

The memory 112 includes non-transitory, computer-readable storage media that stores software that is executed by the processor 116 and which controls the operation of the ventilator 100. In an example, the memory 112 includes one or more solid-state storage devices such as flash memory chips. In an alternative example, the memory 112 may be mass storage connected to the processor 116 through a mass storage controller (not shown) and a communications bus (not shown). Although the description of computer-readable media contained herein refers to a solid-state storage, the computer-readable storage media can be implemented as any available non-transitory media that can be accessed by the processor 116. That is, computer-readable storage media includes non-transitory, volatile and non-volatile, removable and non-removable media implemented in any method or technology for storage of information such as computer-readable instructions, data structures, program modules or other data. For example, computer-readable storage media includes RAM, ROM, EPROM, EEPROM, flash memory or other solid-state memory technology, CD-ROM, DVD, or other optical storage, magnetic cassettes, magnetic tape, magnetic disk storage or other magnetic storage devices, or any other medium which can be used to store the desired information and which can be accessed by the computer.

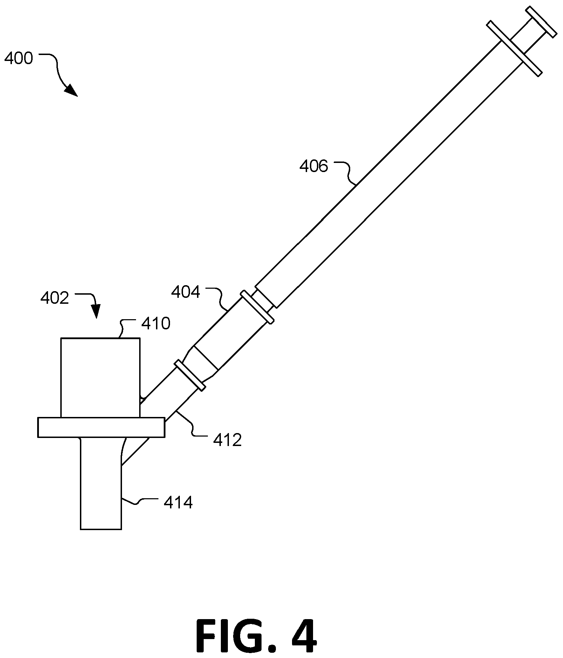

FIG. 1 also shows a humidification system 118 and a fluid delivery system 124. Aspects of the humidification system 118 and the fluid delivery system 124 are further described herein at least with respect to FIGS. 4A-4B, 7, 8A-8B, and 9A-9D. Although the humidification system 118 and the fluid delivery system 124 are shown as two separate systems, the systems may be integrated or components of the systems may be shared (e.g., a shared nozzle, pump, valve, controller, etc.). Additionally, although the humidification system 118 and the fluid delivery system 124 are shown interacting with specific components of the ventilator 100 (e.g., the ventilation tubing system 130 and the patient interface 180), the humidification system 118 or the fluid delivery system 124 may be located in other positions along the breathing circuit or ventilation tubing system 130 (e.g., along the inhalation limb 132, at the wye 170, or downstream of the wye 170).

Communication between components of the ventilator system or between the ventilator system and other therapeutic equipment and/or remote monitoring systems may be conducted over a distributed network, as described further herein, via wired or wireless means. Further, the present methods may be configured as a presentation layer built over the TCP/IP protocol. TCP/IP stands for "Transmission Control Protocol/Internet Protocol" and provides a basic communication language for many local networks (such as intra- or extranets) and is the primary communication language for the Internet. Specifically, TCP/IP is a bi-layer protocol that allows for the transmission of data over a network. The higher layer, or TCP layer, divides a message into smaller packets, which are reassembled by a receiving TCP layer into the original message. The lower layer, or IP layer, handles addressing and routing of packets so that they are properly received at a destination.

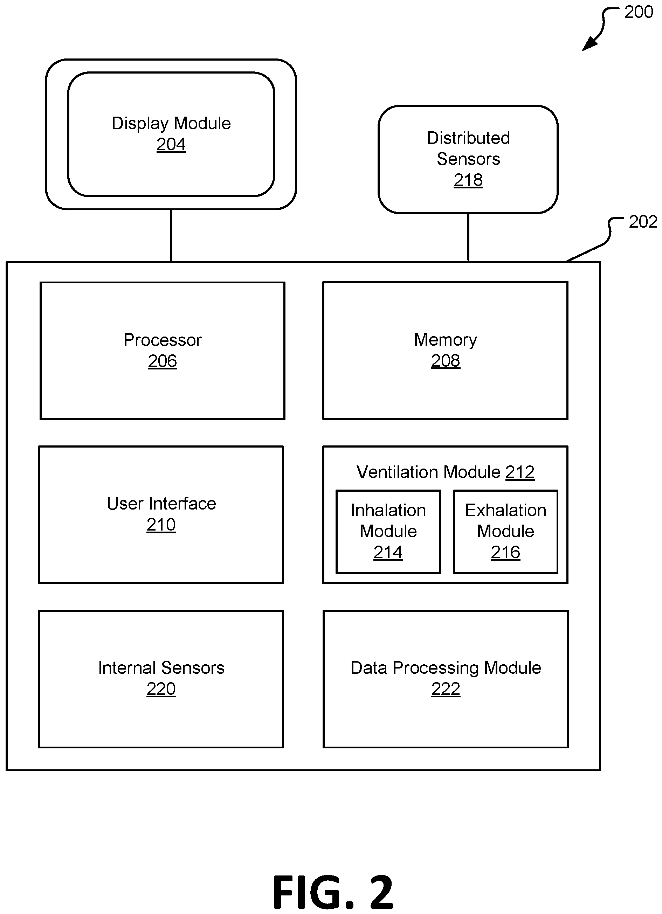

FIG. 2 is a block-diagram illustrating an example of a ventilator system 200. Ventilator system 200 includes ventilator 202 with various modules and components. That is, ventilator 202 may further include, among other things, memory 208, one or more processors 206, user interface 210, and ventilation module 212 (which may further include an inhalation module 214 and an exhalation module 216). Memory 208 is defined as described above for ventilation module 212. Similarly, the one or more processors 206 are defined as described above for one or more processors 206. Processors 206 may further be configured with a clock whereby elapsed time may be monitored by the ventilator system 200.

The ventilator system 200 may also include a display module 204 communicatively coupled to ventilator 202. Display module 204 provides various input screens, for receiving input, and various display screens, for presenting useful information. Inputs may be received from a clinician. The display module 204 is configured to communicate with user interface 210 and may include a graphical user interface (GUI). The GUI may be an interactive display, e.g., a touch-sensitive screen or otherwise, and may provide various windows (i.e., visual areas) comprising elements for receiving user input and interface command operations and for displaying ventilatory information (e.g., ventilatory data, alerts, patient information, parameter settings, modes, etc.). The elements may include controls, graphics, charts, tool bars, input fields, icons, etc. Alternatively, other suitable means of communication with the ventilator 202 may be provided, for instance by a wheel, keyboard, mouse, or other suitable interactive device. Thus, user interface 210 may accept commands and input through display module 204, such as a target humidity or a desired volumetric dosage of medicine. Display module 204 may also provide useful information in the form of various ventilatory data regarding the physical condition of a patient and/or a prescribed respiratory treatment. The useful information may be derived by the ventilator 202, based on data collected by a data processing module 222, and the useful information may be displayed in the form of graphs, wave representations (e.g., a waveform), pie graphs, numbers, or other suitable forms of graphic display. For example, the data processing module 222 may be operative to determine ventilation settings (otherwise referred to as ventilatory settings, or ventilator settings, or ventilation settings) associated with a target humidity or a desired volumetric dosage of medicine, display information regarding the humidity or volumetric dosage of medicine delivered or desired, or may otherwise use humidity and/or medicine dosage in connection with the ventilator, as detailed herein.

Ventilation module 212 may oversee ventilation of a patient according to ventilation settings. Ventilation settings may include any appropriate input for configuring the ventilator to deliver breathable gases to a particular patient, including measurements and settings associated with exhalation flow of the breathing circuit (e.g., measurements which may be taken by internal sensors 220 or distributed sensors 218). Ventilation settings may be entered, e.g., by a clinician based on a prescribed treatment protocol for the particular patient, or automatically generated by the ventilator, e.g., based on attributes (i.e., age, diagnosis, ideal body weight, predicted body weight, gender, ethnicity, etc.) of the particular patient according to any appropriate standard protocol or otherwise, such as may be determined in association with a target humidity, desired medication dosage, pump control, etc. In some cases, certain ventilation settings may be adjusted based on the exhalation flow, e.g., to adjust or improve the prescribed treatment. Ventilation settings may include inhalation flow, frequency of delivered breaths (e.g., respiratory rate, (*f*), tidal volume (V_{T}), PEEP level, etc.).

Ventilation module 212 may further include an inhalation module 214 configured to deliver gases to the patient and an exhalation module 216 configured to receive exhalation gases from the patient, according to ventilation settings that may be based on the exhalation flow. As described herein, inhalation module 214 may correspond to the inhalation module 104, or may be otherwise coupled to source(s) of pressurized gases (e.g., air, oxygen, and/or helium), and may deliver gases to the patient. As further described herein, exhalation module 216 may correspond to the exhalation module 108, or may be otherwise coupled to gases existing the breathing circuit.

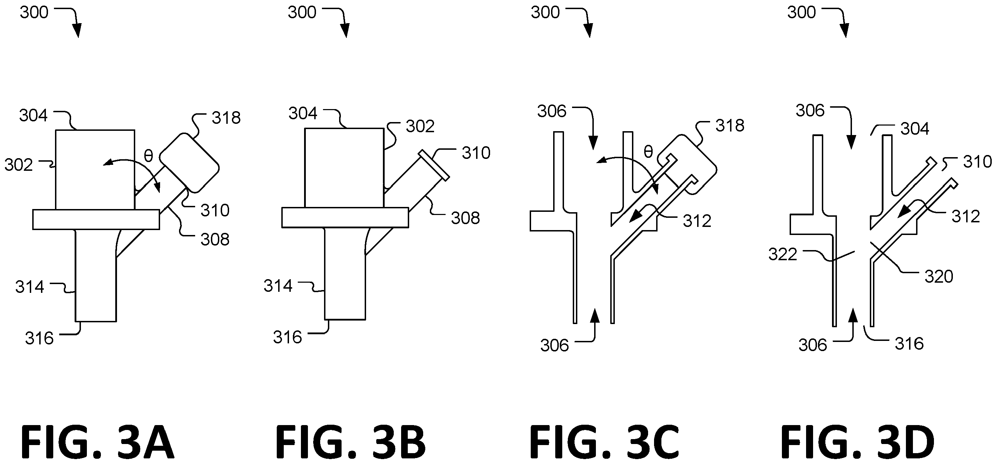

FIGS. 3A-3D show different views of a connector 300 for coupling ventilation tubing to an endotracheal tube. The connector 300 is shown in several views, including a capped connector 300 with a cap 318 in FIGS. 3A and 3C, and an uncapped connector 300 without the cap 318 in FIGS. 3B and 3D. FIGS. 3A-3B show a side view of the connector 300, and FIGS. 3C-3D show a cutaway view of the connector 300. The connector 300 may form a portion of, or be integrated into, an invasive interface, such as an endotracheal tube. In other examples, the connector 300 may be separate or removable from the invasive interface.

The connector 300 includes a primary port 302 with a primary receiving end 304, a secondary port 308 with a secondary receiving end 310, and a patient-side port 314 with a patient-side end 316. A primary channel 306 extends through the primary port 302 and the patient-side port 314 between the primary receiving end 304 and the patient-side end 316. Additionally, a secondary channel 312 extends through the secondary port 308 between the secondary receiving end 310 and a junction end 320 fluidly coupling the secondary channel 312 with the primary channel 306 at a junction 322.

In FIGS. 3A and 3C, the connector 300 is shown with a cap 318 closing the secondary receiving end 310 of the secondary port 308 of the connector. Alternatively, in FIG. 3B and 3D, the connector 300 is shown without the cap 318, such that the secondary receiving end 310 of the secondary port 308 is open and ready to receive an injection tip as discussed further below. When the cap 318 is coupled to the connector 300, the cap 318 blocks the secondary channel 312 and contains or isolates the primary channel 306 from exposure to room air via the secondary port 308. For instance, gases flow into the primary receiving end 304 of the primary channel 306 and out of the patient-side end 316 of the primary channel 306 during an inhalation phase. During an exhalation phase, gases flow into the patient-side end 316 of the primary channel 306 and out of the primary receiving end 304 of the primary channel 306, with no other gas exiting or entering the connector 300 via the secondary channel 312. The cap 318 prevents the delivered breathing gases and/or the exhaled gas from the patient from exiting the secondary port 308.

When the cap 318 is removed from the connector 300, gases or fluids may enter or exit the connector 300 via the secondary channel 312 (e.g., via the secondary receiving end 310 or the junction end 320), in addition to gases entering the connector 300 via the primary channel (e.g., via the primary receiving end 304 or the patient-side end 316). Additionally, when the cap 318 is removed from the connector 300, gases entering or exiting the secondary channel 312 (e.g., via the secondary receiving end 310 or the junction end 320) merge into or split from the primary channel 306 around a junction 322 of the primary channel 306 and the secondary channel 312.

The primary port 302 may be removably couplable with ventilation tubing at the primary receiving end 304 of the primary channel 306. When coupled with the ventilation tubing, the connector 300 is positioned downstream of a wye (e.g., wye 170) in a breathing circuit. The breathing circuit provides flow of breathing gases to a patient via the primary channel 306, as provided via the ventilation tubing coupled to the primary receiving end 304. The primary channel 306, as shown, extends in a substantially straight path through the connector 300 to the patient-side end 316 of the patient-side port 314.

The secondary port 308 is configured to receive an injection tip (e.g., injection tip 404, 500, 704, described below) at the secondary receiving end 310 of the secondary channel 312. The secondary channel 312 extends along a substantially straight path between the secondary receiving end 310 and a junction end 320. The secondary channel 312 provides a direct line into primary channel 306, which is fluidly coupled with a patient's airway or lungs. For example, as described above, during an inhalation phase of the patient, flow of breathing gases is provided to the patient through the primary channel 306 in a direction from the primary receiving end 304 to the patient-side end 316. During an exhalation phase of the patient, exhalation gases flow from the patient-side end 316 to the primary receiving end 304. When the secondary receiving end 310 is capped or coupled with an injection tip (as further described below), flow of breathing gases or flow of exhalation gases are prevented from escaping the connector 300 via the secondary receiving end 310.

The patient-side port 314 is positioned proximate a patient. For example, the patient-side port 314 is positioned proximate a mouth or nose of a patient. The patient-side end 316 is integrated into, or configured to couple, to a patient interface, such as an endotracheal tube, to fluidly couple the breathing circuit with the patient via the connector 300.

The secondary channel 312 intersects with the primary channel 306 at an angle θ, relative to the flow of breathing gases through the primary channel 306. The angle θ may be defined as the angle between a central axis of the primary channel 306 and a central axis of the secondary channel 312. As shown, the angle θ also describes the angle between the primary port 302 and the secondary port 308. The angle θ is less than or equal to 90 degrees. In an example, the angle θ is less than 90 degrees such that a flow vector of the breathing gases through the primary channel 306 shares a common directional component with a flow vector of a fluid injected into the secondary channel 312 at the secondary receiving end 310. Described alternatively, the secondary channel 312 is at an angle θ, relative to the primary channel 306, such that flow through the secondary channel 312 from the secondary receiving end 310 is not opposite or directly orthogonal to a flow delivered to a patient (e.g., a flow of breathing gases delivered during an inhalation phase). By having the angle θ be less than 90 degrees, aerosolized fluid injected into the secondary port 308 is more easily carried by the airflow in the primary channel 306 into the patient's lungs.

FIG. 4 shows a fluid delivery system 400. The fluid delivery system 400 includes a connector 402, an injection tip 404, and a pressure applicator 406. The connector 402 may have similar features as the connector 300 described above with respect to FIGS. 3A-3D. For example, the connector 402 includes a primary port 410, a secondary port 412, and a patient-side port 414. The primary port 410 may couple with ventilation tubing extending toward a wye (e.g., wye 170 in FIG. 1) of a breathing circuit. The secondary port 412 may couple with an injection tip 404. The injection tip 404 may provide aerosolized fluid into the secondary port 412 of the connector 402, as otherwise described herein. Additionally, when not coupled with the injection tip 404, a removable cap (e.g., cap 318 in FIGS. 3A and 3C) may close off the secondary port 412 of the connector 402. The patient-side port 414 may removably couple with an endotracheal tube or other patient interface leading to an airway of a patient.

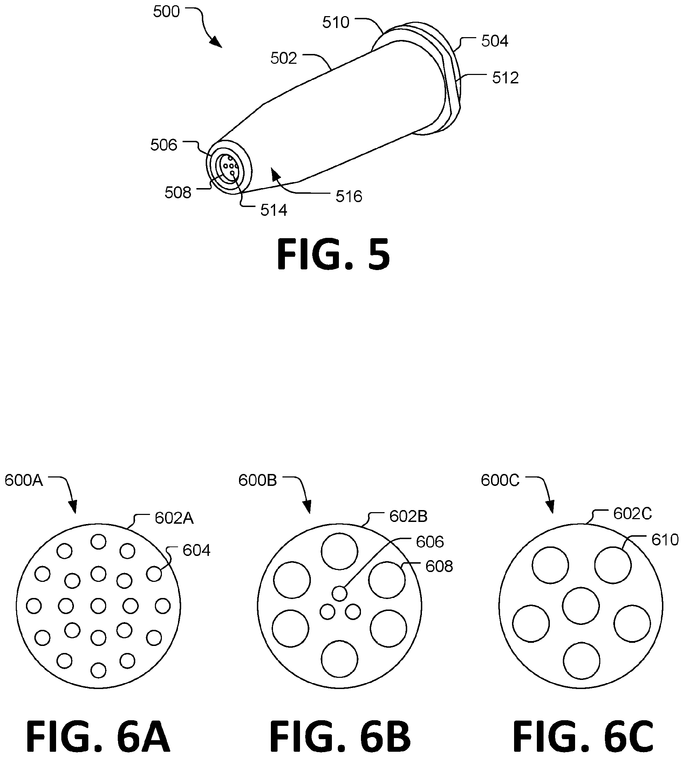

The injection tip 404 may be removably coupled to the secondary port 412 of the connector 402. For instance, the injection tip 404 may be removed and replaced with a different injection tip. In other examples, the injection tip 404 may be permanently attached to the secondary port 412. Additionally, the injection tip 404 removably couples with a pressure applicator 406 to provide a fluid coupling between the pressure applicator 406 and the connector 402. Features of the injection tip 404 are further described below at least with respect to FIG. 5.

The pressure applicator 406 is configured to inject a fluid, such as water or medicine, into the injection tip 404. When injecting the fluid into the injection tip 404, the pressure applicator 406 pressurizes the fluid against a membrane having a set of orifices. Additionally, when the pressure applicator 406 injects the fluid into the injection tip 404, the injection tip 404 is configured to cause the fluid to be aerosolized (e.g., dispersed into a fine spray or tiny droplets). The aerosolized fluid is sprayed into the connector 402 via the secondary port 412 when the pressure applicator 406 is actuated. The pressure applicator 406 may be manually or mechanically actuated. In the example shown, the pressure applicator 406 is a syringe that is actuated manually. Alternatively, the pressure applicator 406 may include a pump actuated electro-mechanically (as otherwise described herein).

FIG. 5 shows an example injection tip 500. The injection tip 500 is configured to removably couple with a connector (e.g., connector 300) at a port (e.g., secondary port 308). The example injection tip 500 includes a body 502, a first end 504, a second end 506, a membrane 508, a flange 510, and a tapered portion 516.

The body 502 of the injection tip 500, as shown, is substantially elongate and extends from the first end 504 to the second end 506. The body 502 is hollow and includes an injection channel extending between the first end 504 and the second end 506. A flange 510 is positioned proximate the first end 504. The flange 510 extends radially outward from the body 502 of the injection tip 500. The flange 510 may include a notch 512. The position of the notch 512 may provide orientation information about the injection tip 500. In an example, the notch 512 may assist in rotationally orienting a pressure applicator to be coupled to the first end 504 of the body 502 of the injection tip 500.

The body 502 may also include a tapered portion 516 proximate the second end 506. The tapered portion 516 may facilitate coupling of the second end 506 with a channel of a connector (e.g., secondary channel 312 of connector 300). The tapered portion 516 may have a smaller diameter closer to the second end 506 and a larger diameter closer to the first end 504. In such an example, the tapered portion 516 may have a frustoconical shape.

The membrane 508 is positioned inside and across the injection channel of the injection tip 500. In the example shown, the membrane 508 is positioned proximate the first end 504 of the injection tip 500 and orthogonal to the injection channel such that flow through the channel is restricted by the membrane 508. The membrane 508 may be a plate having a circumference that matches the interior of the body 502. The membrane 508 may be made of the same or different type of material as the body 502, such as a metal, plastic, or composite material. The material of the membrane 508 and/or the body 502 may be a rigid, durable material that is able to withstand high pressures of fluid, such as pressures in excess of 250 pounds per square inch (PSI), 300 PSI, and/or 350 PSI. The membrane 508 includes a set of orifices 514. The set of orifices 514 includes at least one orifice through which a fluid may cross, or pass through, the membrane 508.

The orifice(s) 514 may be sized based on a target droplet size, a fluid flow property (e.g., viscosity, density, etc.) of a fluid to be pushed through the set of orifices of the membrane 508, and/or a pressure to be applied to the fluid at the membrane 508. The set of orifices 514 are sized and positioned about the membrane 508 to cause a fluid to aerosolize into a spray or mist of droplets when pushed through the set of orifices across the membrane 508. At constant pressure, a fluid with a higher viscosity may be paired with a larger set of orifices to achieve a similar particle size to that of a fluid with a lower viscosity paired with a smaller set of orifices. At constant viscosity, a higher pressure applied to a fluid may be paired with a smaller set of orifices to achieve a similar particle size to that of a lower pressure applied to a fluid paired with a larger set of orifices. The pressure applied may vary based on the pressure applicator and/or components of the pressure applicator (e.g., a manually actuated syringe, a size and speed of a pump, etc.). Thus, sizing of the set of orifices may be selected based on a fluid property of the fluid being aerosolized through the membrane 508 and a pressure at which the fluid is to be pushed through the membrane 508. The number of orifices 514 may include at least 4, 6, or 8 orifices in some examples.

The injection tip 500 may be removable, disposable, customizable, and/or configurable. For example, the injection tip 500 may be removably coupled with a pressure applicator at the first end 504 and removably coupled with a connector at the second end 506. The injection tip 500 may also be disposable and/or replaceable. For example, the injection tip 500 may be switched out between uses, between patients, or between different fluids being aerosolized by the injection tip 500. For instance, a first injection tip 500 with a first orifice configuration (e.g., number of orifices, size of orifices, etc.) may be used for delivery of a first fluid (e.g., a first medicine), and a second injection tip with a second orifice configuration may be used for delivery of a second fluid (e.g., a second medicine) having different fluid characteristics than the first fluid. Additionally, removability, disposability, and/or replaceability of the injection tip 500 may allow a clinician to easily access, inspect, clean, and or change the injection tip 500, when required or desired.

The injection tip 500 may be customizable in appearance (e.g., color, shape, marking, etc.) for ease of selection by a clinician. For example, an injection tip 500 may be customized based on fluid properties of a fluid to be aerosolized via the injection tip 500 (e.g., an injection tip 500 may be a first color for fluids with a first range of viscosities and a second color for fluids with a second range of viscosities). Additionally or alternatively, customizable features of the injection tip 500 may be based on specific medications. For instance, an appearance of an injection tip 500 may be based on a configuration of the membrane 508 and/or the set of orifices 514 in the injection tip 500.

The injection tip 500 may also be configurable. For example, the injection tip 500 may have multiple configurations associated with different sizing and/or positioning of the set of orifices 514. In an instance, the membrane 508 of the injection tip 500 may be switchable. In another instance, different rotational orientations of the injection tip 500 (which may be indicated by a notch 512) may be associated with different configurations of the set of orifices 514. In an instance, a rotation of the injection tip (e.g., relative to a pressure applicator or connector) may cause a change in size of one or more orifices of the set of orifices 514 or may cause one or more orifices of the set of orifices 514 to be opened or closed. Different configurations, sizing, and positioning of the set of orifices 514 is further described below with respect to FIGS. 6A-6C.

FIGS. 6A-6C show different configurations 600A, 600B, 600C for a set of orifices on a membrane 602A, 602B, 602C of an injection tip. FIG. 6A shows relatively smaller sized orifices 604 of the set of orifices on the membrane 602A. FIG. 6B shows small orifices 606 and relatively large orifices 608 included in the set of orifices on the membrane 602B. FIG. 6C shows relatively larger sized orifices 610 of the set of orifices on the membrane 602C. Although FIGS. 6A-6C show membranes 602A, 602B, 602C with a specific quantity of orifices (e.g., as shown, 21, 9, and 6, respectively), any number of orifices may be implemented (e.g., 50 or less, 25 or less, 10 or less, etc.). The orifices of the set of orifices may be spaced radially or axially about the membrane and/or cross section of the inner cavity of the injection tip. Additionally, the orifices of the set of orifices may be spaced symmetrically about one or more halves of the membrane and/or cross section of the inner cavity of the injection tip. Laser drilling or cutting, stamping, or other techniques for generating micro-holes of the sizes discussed herein may be used to create the orifices in the membrane.

Unlike traditional nebulizers, the present membrane 602A-602C and orifices do not require or utilize vibration to aerosolize fluid. For example, nebulizers often utilize a mesh that is placed in the fluid and vibrated at a high frequency. The vibrating mesh causes the fluid to form a mist of the fluid. In contrast, the present technology utilizes a high pressure of fluid through a stationary membrane with orifices. As the pressurized fluid passes through the orifices, the fluid is aerosolized. Accordingly, the present technology does not require any moving parts in the injection tip itself, which may provide for a more consistent and reliable delivery of the fluid. Moreover, the injection tip does not have to be powered.

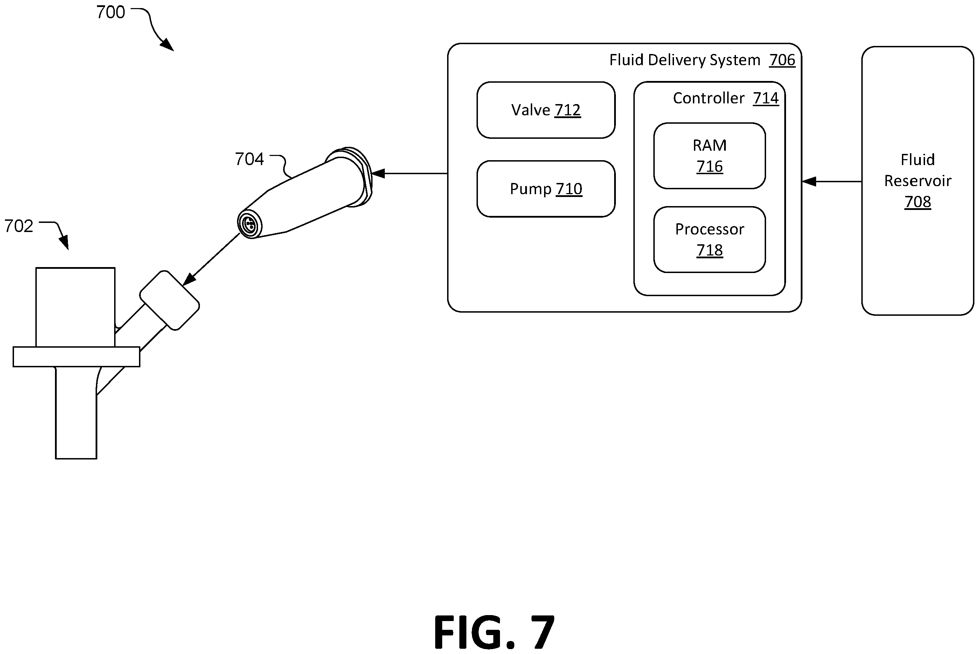

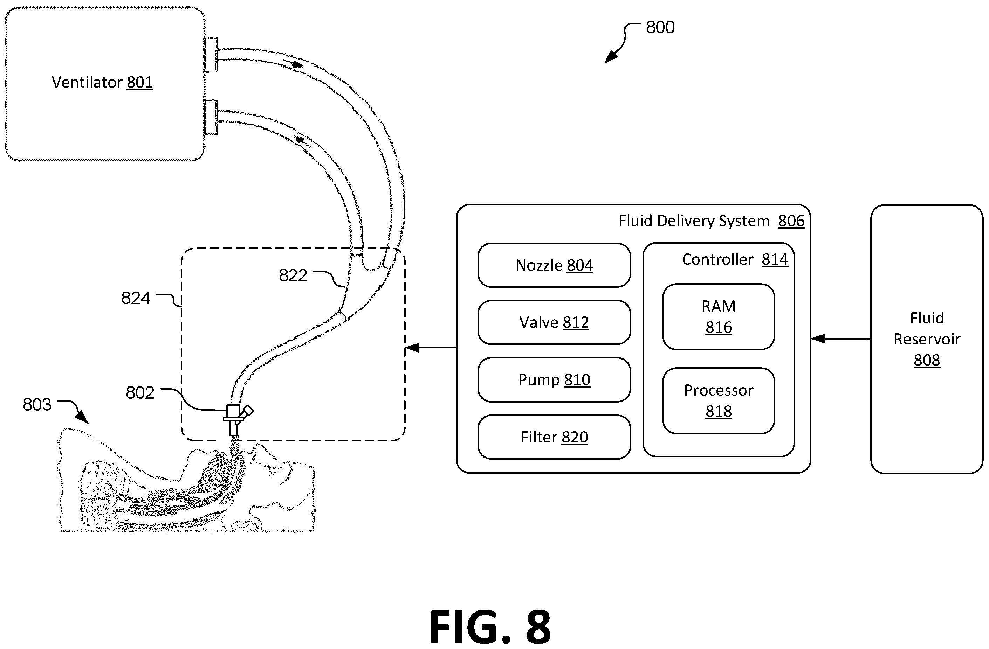

FIG. 7 shows a system 700 for delivering aerosolized fluid. The system 700 includes a connector 702, an injection tip 704, a fluid delivery system 706, and a fluid reservoir 708. The connector 702 may have similar features to the connector 300 described with respect to FIGS. 3A-3D, above. The injection tip 704 may have similar features to the injection tip 500 described with respect to FIG. 5, above. Although the injection tip 704 is shown as a separate component from the fluid delivery system 706, the injection tip 704 may otherwise be referred to herein as a component of the fluid delivery system 700 (e.g., such as nozzle 804 in FIG. 8, described below). A nozzle that is included in lieu of the injection tip 704, as a component of the fluid delivery system 706, may have some features different from the injection tip 704 (e.g., may not be removable and/or disposable).

The connector 702 of the system 700 may be coupled to ventilation tubing (e.g., at a primary receiving end of a primary channel) and an endotracheal tube (e.g., at a patient-side end of the primary channel). The injection tip 704 is configured to be removably coupled with the connector 702 (e.g., at a secondary receiving end of a secondary channel) and the fluid delivery system 706. In an example, the fluid delivery system 706 is coupled to the injection tip 704 via tubing.

The fluid delivery system 706 shown in FIG. 7 is electro-mechanically actuated and controlled by a controller 714. The fluid delivery system 706 pressurizes and delivers fluid from the fluid reservoir to the injection tip 704. The fluid may be pressurized by a pump 710. The pressurized fluid is pushed through a membrane of the injection tip 704 (e.g., membrane 508, 602A, 602B, 602C) to aerosolize the fluid into a mist or spray, which is provided into the connector through a secondary channel to be mixed with breathing gases flowing through the primary channel of the connector for delivery to the patient.

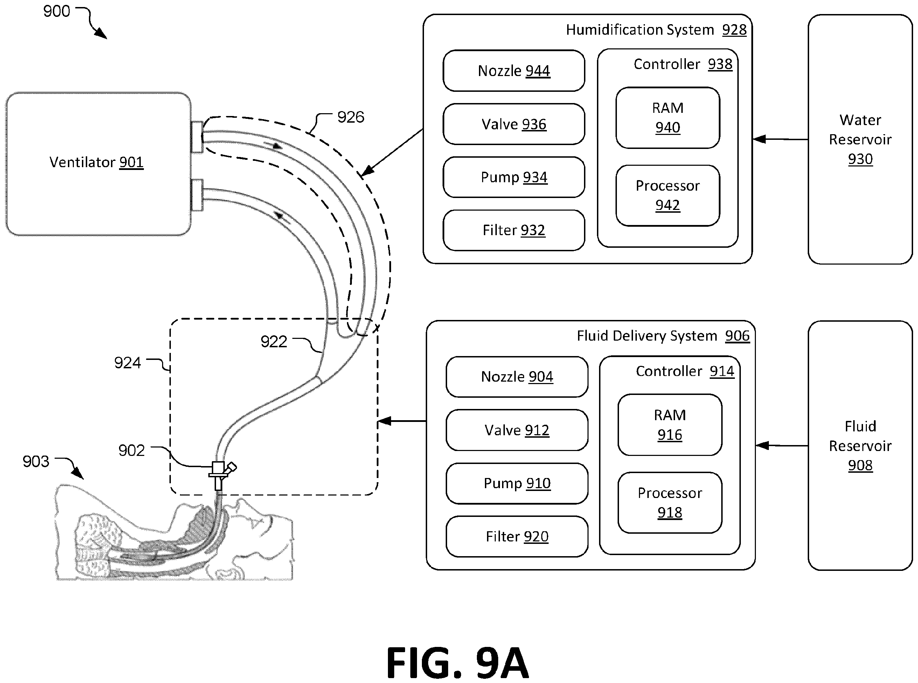

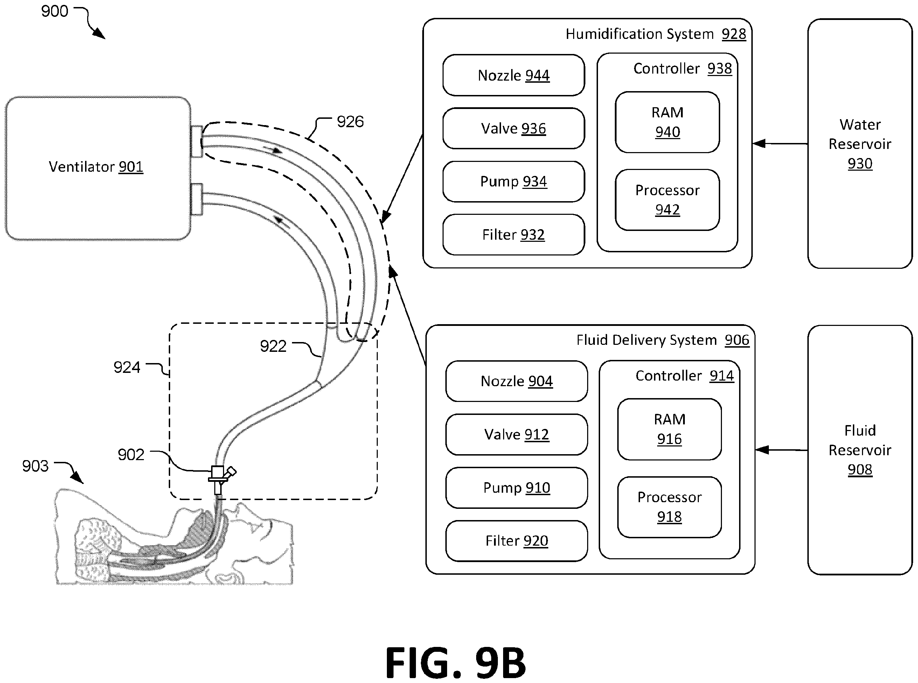

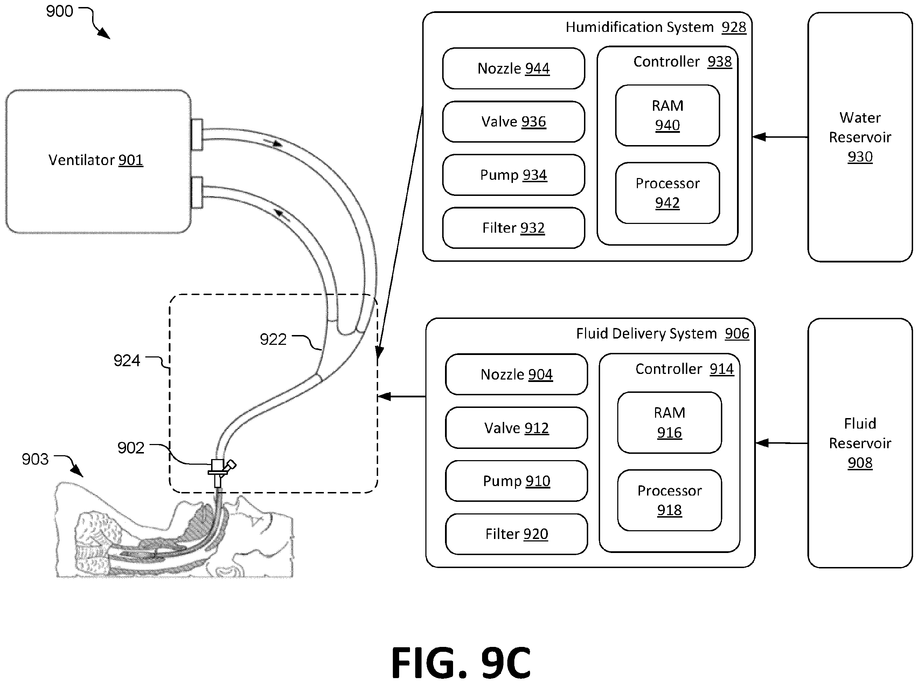

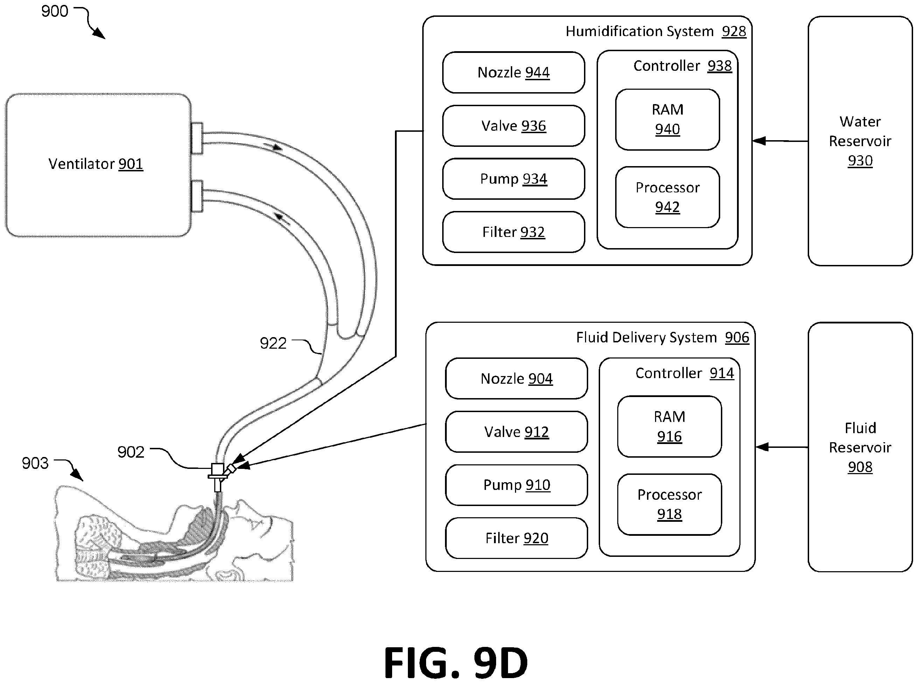

The example fluid delivery system 706, as shown in FIG. 7, includes a pump 710, a valve 712, and a controller 714. The pump 710 may be a high-pressure pump 710. The pump 710 pressurizes and delivers fluid from the fluid reservoir 708 through the valve 712 (e.g., a one-way valve, which may be optional) to the injection tip 704. The pump 710 and/or valve 712 may be controlled by the controller 714. The controller 714 includes memory 716 and at least one processor 718. Control of the pump 710 by the controller 714 is further described below at least with respect to FIGS. 8 and 9.

The system 700 introduces volumetric delivery of a fluid (e.g., medication or drug) into breathing gases to be delivered to a ventilated patient. Volumetric delivery of the fluid may be provided to an intubated patient via the system 700 by injecting the aerosolized fluid with a pump 710 of the fluid delivery system 706 directly into the breathing circuit at the connector, which may be positioned proximate an endotracheal tube.

The present systems and methods allow for a controlled delivery of a high-pressure fluid at or adjacent to a patient and/or patient interface. By injecting the medicine after the wye and proximate to the endotracheal tube, rather than between the inspiratory port and the wye, the likelihood that the medicine will be delivered to the patient's lungs is increased. For instance, the medicine is effectively injected into the endotracheal tube and there is less of chance for the medicine to stick to the walls of ventilation tubing of the breathing circuit. In addition, when the injection point is positioned downstream of the wye, the injected medicine is carried to the patient and may not be carried into the expiratory limb or expiratory port of the ventilator, where the medicine may clog an expiratory filter and/or may be wasted. Where medicine is in short supply, conservation of such medicine and a higher confidence of a full-dosage delivery to a patient can be of the utmost importance.

FIG. 8 shows an example ventilation system 800. The example ventilation system 800 includes a ventilator 801 with a breathing circuit connected to a patient 803, a fluid delivery system 806, and a fluid reservoir 808. The fluid delivery system 806 may include a nozzle 804, a pump 810, a valve 812, a controller 814, and a filter 820. The nozzle 804 may be similar to the injection tip 500 described herein. Alternatively, the nozzle 804 may be permanently or semi-permanently integrated into the fluid delivery system 806. In an instance where the nozzle 804 is integrated into the fluid delivery system 806, a membrane of the nozzle 804, having a set of orifices, may be configurable or switchable to allow the fluid delivery system 806 to accommodate fluids (e.g., contained in the fluid reservoir 808) of different fluid flow properties (e.g., viscosity, density, etc.) and/or at different pressures (e.g., as applied by the pump 810). Additionally, in an example where the nozzle 804 is integrated into the fluid delivery system 806, the fluid delivery system 806 may include a connection interface enclosing the nozzle 804, with the connection interface couplable to ventilation tubing of a breathing circuit.

The pump 810 of the fluid delivery system 806 is capable of pressurizing a fluid contained in the fluid reservoir 808 and directing the pressurized fluid into the nozzle 804 to cause aerosolization of the fluid. The pump 810 may be a high-pressure pump. In an example, the pump 810 may deliver the fluid to the nozzle 804 at pressures in excess of 250 PSI, 300 PSI, and/or 350 PSI.

The valve 812 may be positioned between the pump 810 and the nozzle 804 to control release of the fluid into the nozzle 804 and/or to prevent backflow of the fluid into the pump 810. In an example, the valve 812 is a solenoid valve. The filter 820 may filter the fluid from the fluid reservoir 808 before the fluid enters the pump 810 (e.g., the filter is positioned upstream of the pump 810). The filter 820 may prevent contaminants or particles above a certain size from entering the pump 810 and the nozzle 804.

The fluid delivery system 806 may be fluidly coupled to the breathing circuit via coupling of the nozzle 804 and the breathing circuit. The coupling of the nozzle 804 and the breathing circuit may be downstream 824 of the wye 822. By aerosolizing the fluid in breathing gases downstream 824 in the breathing circuit, the fluid is introduced proximate to the patient. Introduction of the aerosolized fluid closer to the patient may result in better accuracy in dosing of the fluid (e.g., how much of the aerosolized fluid reaches the patient). Additionally, the nozzle 804 may be positioned proximate a flow of breathing gases in the breathing circuit to facilitate aerosolization of the fluid in the flow breathing gases and to otherwise reduce rainout of the fluid, reduce likelihood of a stagnant zone of the aerosolized fluid, and/or reduce other barriers preventing aerosolized fluid from mixing with the breathing gases for delivery to the patient 803.

As shown in FIG. 8, the controller 814 of the fluid delivery system 806 includes memory 816 and at least one processor 818. The controller 814 is operative to control the pump 810 (e.g., turning the pump on and off, controlling a speed of the pump, etc.), the valve 812 (e.g., opening or closing the valve for specified durations), and/or the nozzle 804 (e.g., controlling a configuration of a set of orifices on a membrane of the nozzle 804). The controller 814 may be operative to receive commands or measurements from other controllers in the ventilation system 800 (e.g., a controller of the ventilator and/or a controller associated with other delivery systems, such as humidification system 928 in FIGS. 9A-9D). In an example, the controller 814 of the fluid delivery system may receive an inspiratory flow command from the ventilator 801 and control the pump 810, valve 812, and/or nozzle 804 to cause aerosolization of a fluid for a specified duration at a specified time (e.g., relative to a breath phase of a patient, a flow of breathing gases, a humidity of breathing gases, etc.). In other examples, the fluid delivery system 806 may include one or more flow sensors, which sense or measure the flow of breathing gases at the location of the nozzle 804 and/or in the breathing circuit. The one or more flow sensors may also determine directionality of the flow to indicate whether gases are being delivered to the patient or exhaled from the patient. In examples where the fluid delivery system 806 includes the flow sensors, the fluid delivery system may not be in communication with the ventilator (e.g., the fluid delivery system 806 may be a standalone system). The amount of aerosolized fluid delivered may be calculated to deliver a user-selected volumetric dosage of the fluid over a user-selected duration, which may include delivering aerosolized fluid over multiple durations provided phasically (e.g., during an inhalation phase, but not during an exhalation phase) over one or more breaths for the user-selected duration. For the purposes of this disclosure, a "breath" refers to a single cycle of inhalation and exhalation delivered with the assistance of a ventilator.

The fluid delivery system 806 may therefore control volumetric dosage aerosolized into the breathing circuit, timing of when the volumetric dose is delivered, and a size of the fluid droplets aerosolized. Control over volumetric dosage, timing of doses, and/or droplet size may be beneficial to treating a patient. For example, volumetric dosage may be prescribed based on a weight of the patient, and adherence to the prescription may impact the health of the patient. Additionally, timing of dose delivery and particle size may influence absorption of the fluid into the lungs of the patient. This may be, in part, due to non-uniform filling of the lungs during an inhalation phase of a breath. For instance, aerosolization of a fluid during a first half of an inhalation phase may draw the fluid deeper into the lungs of the patient than aerosolization during a second half of the inhalation phase of the same breath. Additionally, smaller particle sizes may be drawn deeper into the lungs than larger particle sizes or may impact absorption once inside the lungs.

The efficiency of the volumetric dose that is delivered to the patient, as compared with the volumetric dosage that is introduced into the breathing circuit, increases as the aerosolization is moved closer to the patient. Greater efficiency of volumetric dosage delivery results in increased accuracy of volumetric dosage delivered to the patient and a reduction in fluid accumulating in an exhalation port of the ventilator 801 or contaminating other components of the ventilator 801 (e.g., an exhalation flow sensor). Thus, placement of the nozzle 804 of the fluid delivery system 806 proximate to the patient may be desirable. In the example shown in FIG. 8, the nozzle 804 of the fluid delivery system 806 is positioned downstream 824, such as at the wye 822, at a connector 802 coupled to an endotracheal tube, or in between the wye 822 and the connector 802.

By delivering a volumetric dose, the fluid delivery system 806 may streamline the patient treatment process because medicines and drugs are often prescribed in volume per weight of the patient. In this way, no other calculations are needed to determine if a proper dose is administered to the patient, assuming the efficiency of volumetric dosage delivery is known or can be accurately estimated. Further, an evaluation of drug effectiveness is facilitated by the clinician knowing how much of the drug is actually being delivered to the patient (e.g., the efficiency of the volumetric dosage).

The efficiency of volumetric dosage may also be based on timing of delivery of the volumetric dose. The timing of delivery may be phasic for each breath and may be applied over a duration spanning multiple breaths. For example, a volumetric dose may be entirely applied during a single duration period that may span one or more breaths. In another example, the volumetric dose may be applied only during an inhalation phase of one or more breaths (e.g., if applied over multiple breaths, the volumetric dose may include a duration period for each breath accumulating in a total duration during which the volumetric dose is delivered). As further described herein, lungs often fill in a nonuniform manner during an inhalation phase, with gases inhaled at a beginning of the inhalation phase drawn deeper into the lung than gases inhaled towards the end of the inhalation phase. Due to this nonuniform filling of the lungs, a volumetric dose timed during a first half or first two thirds of an inhalation phase may result in greater dosage efficiency than a volumetric dose timed during a second half or last third of the inhalation phase.

Timing of delivery of a volumetric dose may be user-specified. For example, time-based variables that may be user-specified for a volumetric dose include phasic or continuous dosing and/or dose period. Phasic dosing may be based on timing the dose with an inhalation phase or a portion of an inhalation phase. Alternatively, continuous dosing may introduce and maintain a constant dose continuously. Continuous dosing may require a lower rate of aerosolization of fluid into the breathing circuit to increase the likelihood that the fluid reaches the patient 803 (e.g., rather than raining out in the circuit or contaminating other components of the ventilator 801). The dose period is a period of time over which the volumetric dose is to be delivered. As otherwise described herein, multiple partial doses of fluid may be delivered over multiple durations (e.g., during inhalation phases of different breaths during the dose period) to accumulate in delivery of the total volumetric dose over the dose period.

The droplet size of a fluid results from a variety of droplet parameters, including a configuration (e.g., size, spacing, quantity, etc.) of a set of orifices in a membrane, a pressure of the fluid applied to the membrane, and a fluid flow property of the fluid. One or more of these droplet parameters may be adjusted to achieve a desirable droplet size or size range. For example, membrane configuration and/or pressure may be adjusted to result in a droplet size range (e.g., 6-9 microns, 7-11 microns, etc.). Additionally or alternatively, a fluid flow property may be changed, such as by changing the fluid or changing the concentration of the fluid.

The efficiency of volumetric dosage delivery for a given breathing circuit and fluid delivery setup can be tested using simulated lungs. A known efficiency may then be used to determine volumetric dosage delivered to the patient for a given setup. As described above, efficiency of volumetric dosage may be based on the position of the nozzle 804 relative to the patient 803 and the timing of volumetric delivery (e.g., during a first half of an inhalation phase of a breath). In an example where the nozzle 804 of the fluid delivery system is positioned proximate the patient 803 (e.g., at connector 802) and the delivery of the aerosolized fluid is phasic, substantially all of the volumetric dose may be delivered to the patient. Other parameters may also be tested, such as varying fluid flow properties of the fluid, varying pressure at which the fluid is pumped into the nozzle 804, and/or varying orifice configurations on a membrane of the nozzle 804.

FIGS. 9A-9D show different configurations for a ventilation system 900. The ventilation system 900 may include the components described with respect to FIG. 8, in addition to a humidification system 928. For example, the ventilation system 900 includes a ventilator 901 supporting a patient 903, a fluid delivery system 906 coupled to a fluid reservoir 908, and a humidification system 928 coupled to a water reservoir 930. The breathing circuit includes an upstream portion 926 upstream of a wye 922, a downstream portion 924 downstream of and including the wye 922, and a connector 902.

The fluid delivery system 906 may be similar to, and have similar components of, the fluid delivery system 806 described in FIG. 8. For example, the fluid delivery system 906 in FIGS. 9A-9D includes a nozzle 904, a pump 910, a valve 912, a controller 914 with memory 916 and at least one processor 918, and a filter 920. The humidification system 928 may have components that are similar to the components of the fluid delivery system 906, including a filter 932, a pump 934, a valve 936, a nozzle 944, and a controller 938 having memory 940 and at least one processor 942. Although the fluid delivery system 906 and the humidification system 928 are shown as two separate systems in FIGS. 9A-9D, one or more components of the fluid delivery system 906 and the humidification system 928 may be shared, such as a controller 914, 938, pump 910, 934, and/or valve 912, 936. Certain components may not be shared between (e.g., be separate from) the fluid delivery system 906 and the humidification system 928, such as the fluid reservoir 908 and the water reservoir 930 and the filter 920, 932.

Although the configurations of the ventilation system 900 shown in FIGS. 9A-9D contemplate the nozzle 904 of the fluid delivery system 906 being separate from the nozzle 944 of the humidification system 928, a shared nozzle may be implemented in the configurations of the ventilation system 900 shown in FIGS. 9B, 9C, and 9D. In an example where the fluid delivery system 906 and the humidification system 928 share a nozzle, the nozzle and/or a set of orifices on a membrane of the nozzle may be configurable. For instance, during delivery of fluid originating from the fluid reservoir 908 through the shared nozzle, the nozzle may be in a fluid configuration or aerosol configuration (e.g., a full cone atomizer, orifices of a larger size, etc.). Alternatively, during delivery of water originating from the water reservoir 930 through the shared nozzle, the nozzle may be in a water configuration or evaporative configuration (e.g., a hollow cone atomizer, radial dispersion of droplets, orifices of a smaller size, etc.).

In an example where the pumps 910, 934 of the fluid delivery system 906 and the humidification system 928 are shared, but the nozzles 904, 944 are not shared, the shared pump may modulate between nozzles depending on a desired quantity of fluid and water to be delivered to the circuit. In an example, switching between the nozzles 904, 944 may be controlled by turning on and off valves 912, 936 or turning on and off the nozzles 904, 944 (e.g., controlling the configuration of the nozzles to open and close a set of orifices). Thus, delivery of droplets into breathing circuit may alternate between the two nozzles 904, 944. Alternatively, humidification of the breathing circuit may be paused (e.g., via selection of a pause button) for a specified duration prior to or after fluid delivery by the fluid nozzle 904 (e.g., to dry out the air prior to fluid delivery) or during active delivery of the aerosolized fluid via the fluid nozzle 904.

The following discussion of FIGS. 9A-9D contemplates the ventilation system 900 including two nozzles 904, 944 (e.g., a "dual nozzle" system 900). The configurations shown in FIGS. 9A-9D show different coupling positions of the two nozzles to the breathing circuit of the ventilation system 900.

In FIG. 9A, a nozzle 904 of the fluid delivery system 906 is positioned in a downstream portion 924 of the breathing circuit (e.g., at or downstream of the wye 922) and the nozzle 944 of the humidification system 928 is positioned in an upstream portion 926 of the breathing circuit (e.g., upstream of the wye 922). The two nozzles 904, 944 are thus positionable in different regions of the breathing circuit or at opposite ends of the breathing circuit. Placement of the nozzle 904 of the fluid delivery system 906 in the downstream portion 924 may facilitate efficiency of a dosage of fluid delivered to the patient 903, as otherwise described herein. Placement of the nozzle 944 of the humidification system 928 may be desirable proximate the ventilator 901 due to clinician preferences, expected humidifier placement in the room, or reducing breaks in ventilation tubing of the breathing circuit (e.g., ventilation tubing required before and after the nozzle 944 of the humidification system).

In FIGS. 9B, 9C, and 9D, the nozzle 904 of the fluid delivery system 906 is positioned proximate the nozzle 944 of the humidification system 928. For example, FIG. 9B shows the nozzle 904 of the fluid delivery system 906 and the nozzle 944 of the humidification system 928 coupled to the breathing circuit in the upstream portion 926. FIG. 9C shows the nozzle 904 of the fluid delivery system 906 and the nozzle 944 of the humidification system 928 coupled to the breathing circuit in the downstream portion 924. FIG. 9D shows the nozzle 904 of the fluid delivery system 906 and the nozzle 944 of the humidification system 928 coupled to the breathing circuit at the connector 902. Positioning of the nozzles 904, 944 proximate each other in the breathing circuit may allow for one or more components of the fluid delivery system 906 and the humidification system 928 to be shared, reduce interruptions in the breathing circuit, or save space in a room where the ventilation system 900 is located.

The nozzle 904 of the fluid delivery system 906 may be positioned downstream of the nozzle of the humidification system 928. This relative downstream positioning of the nozzle 904 of the fluid delivery system 906 may allow spacing between any heated portions of ventilation tube near or about the nozzle 944 of the humidification system 928 (e.g., which may facilitate evaporation of water droplets in the breathing gases to humidify the breathing gases). Additionally, as described above, placement of the nozzle 904 of the fluid delivery system 906 closer to the patient may be desirable to increase dosage efficiency and reduce contamination of ventilator components.

In a dual nozzle system 900, regardless of any sharing of components between the fluid delivery system 906 and the humidification system 928, the nozzles 904, 944 may alternate or otherwise have coordinated delivery of droplets. For example, humidification of the breathing gases may be paused either during active delivery of an aerosolized fluid or for a specified time period before and/or after delivery of the aerosolized fluid. Alternating delivery may reduce washing out of an aerosolized fluid in the breathing circuit due to humidity of the breathing gases. Additionally, dry air achieved by pausing humidification for a time period prior to delivery of aerosolized fluid may be conducive to aerosolizing the fluid in the breathing gases (e.g., drier air may facilitate suspension of fluid droplets).

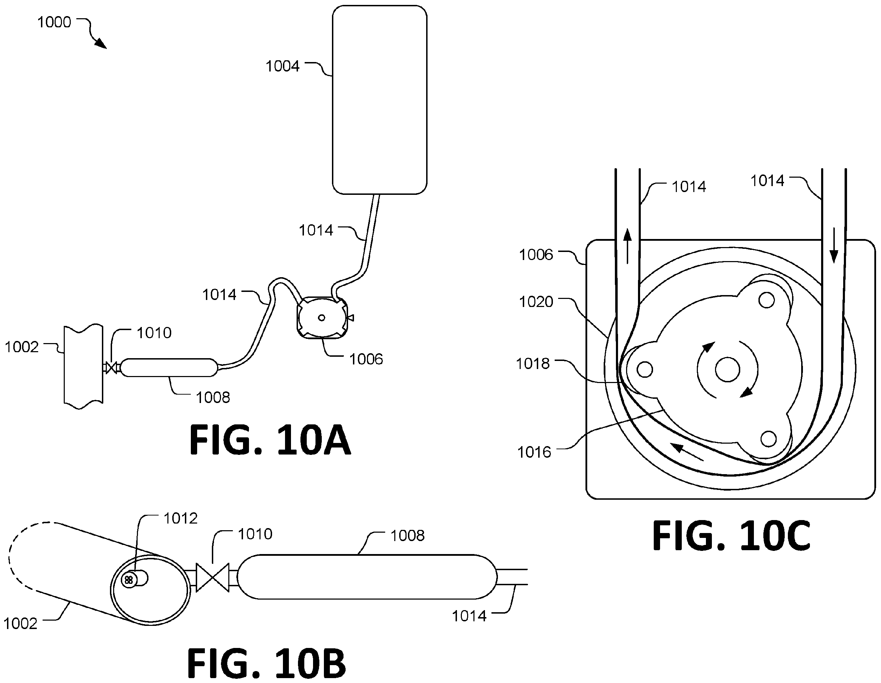

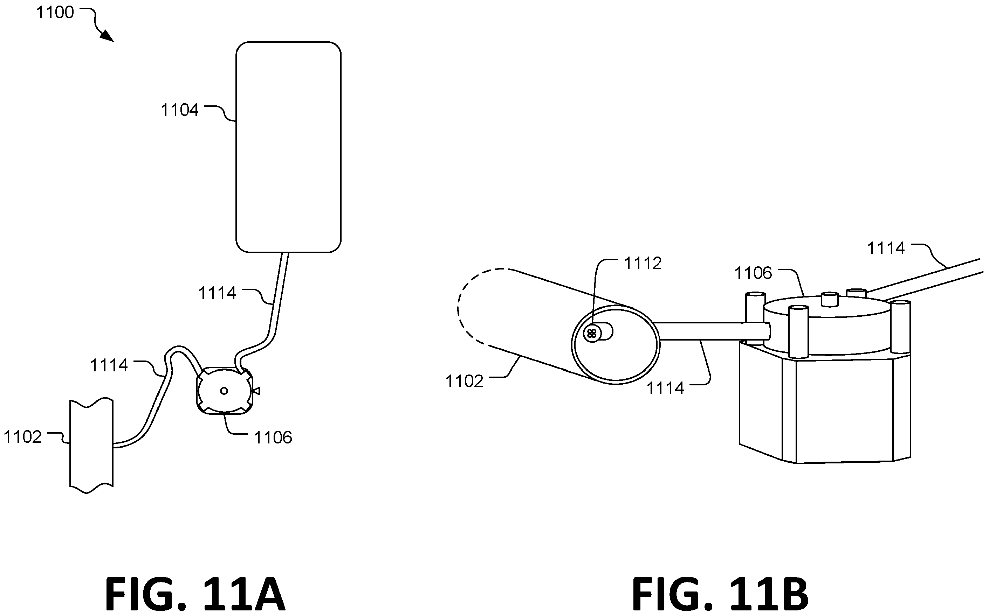

The following discussion of FIGS. 10A-10C and 11A-11B relates to fluid delivery systems 1000, 1100 and their components. In examples, the fluid delivery systems 1000, 1100 may apply to, or be incorporated into, the systems described in FIGS. 7, 8, and 9A-9D. FIGS. 10A-10C show components of an example fluid delivery system 1000. FIGS. 11A-11B show components of another example fluid delivery system 1100.

FIGS. 10A-10C show a fluid delivery system 1000 that injects aerosolized and/or atomized fluid into a breathing circuit 1002 (e.g., injection into breathing circuit tubing, such as along the inhalation limb, at the wye, between the wye and a patient interface, etc., or at the patient interface, such as at an endotracheal tube or mask). The fluid delivery system includes a fluid reservoir 1004, a pump 1006, a pressure chamber 1008, a valve 1010, and a nozzle 1012. A tube or fluid line 1014 fluidly couples the fluid in the fluid reservoir 1004 with the nozzle 1012 for injection into a flow of breathing gases flowing through the breathing circuit 1002. For example, the fluid line 1014 allows fluid to flow from the fluid reservoir 1004 to the pump 1006, to the pressure chamber 1008, to the valve 1010 and into the nozzle 1012 for injection into the breathing circuit 1002.

A wetted path is defined as components of the fluid delivery system 1000 that are in fluidic contact with the fluid flowing from the fluid reservoir 1004 to the nozzle 1012. One of more components of the fluid delivery system 1000 may be outside of the wetted path (e.g., not in fluidic contact with the fluid). For example, the pump 1006 and/or valve 1010 may be positioned outside of the wetted path. Stated alternatively, the fluid reservoir 1004, the pressure chamber 1008, the tubing forming the fluid line 1014, and the nozzle 1012 may exist along the wetted path (e.g., in fluidic contact with the fluid). Components positioned outside of the wetted path may be reusable (e.g., from fluid to fluid, from patient to patient, from day to day, etc.). Further, because the reusable components are outside of the wetted path and are not otherwise contaminating the wetted path or being contaminated by the fluid, the reusable components may not need to be to cleaned, sanitized, or otherwise sterilized prior to reuse.

As stated previously, the pump 1006 may be positioned outside of the wetted path of the fluid delivery system 1000. In the example shown in FIG. 10C, the pump 1006 is capable of pumping fluid through the fluid line 1014 without components of the pump 1006 coming into fluidic contact with the fluid. For instance, the pump 1006 may be a tube pump, such as a peristaltic pump, a full-press ring pump, a mid-press ring pump, or other pump configured to pump a fluid without components of the pump coming into fluidic contact with the fluid. In the example rotary-motion peristaltic pump 1006 shown in FIG. 10C, the components of the pump 1006 may include a rotor 1016 and at least one roller 1018. The fluid line 1014 (e.g., tubing) may feed through the pump 1006 in between the rotor 1016 and a casing 1020. The fluid line 1014 may be removable from the pump 1006 and replaceable with the same or different fluid line (e.g., when a fluid line is contaminated, old, or otherwise desired to be replaced, or if the pump 1006 is moved to a different fluid delivery system 1000). Thus, the pump 1006 may be reusable (e.g., without cleaning or sanitation). Stated differently, the fluid line 1014 may be removed and replaced within the pump 1006, without replacing or cleaning the pump 1006 because the fluid flowing through the fluid line 1014 does not contact the pump 1006 or components of the pump 1006.

As the rotor 1016 of the pump 1006 rotates, a roller 1018 compresses the fluid line 1014 between the roller 1018 and the casing 1020. The compression of the fluid line 1014 by the roller 1018 forces fluid in the fluid line 1014 to flow through the fluid line 1014. As the fluid line 1014 returns to its original shape (e.g., via decompression after the roller 1018 rotates), additional fluid is drawn into the fluid line 1014 (e.g., via peristalsis). Although the peristaltic pump in FIG. 10C shows a rotary-motion peristaltic pump, a linear peristaltic pump may be implemented. Additionally, although three rollers 1018 are shown in FIG. 10C, any number of rollers 1018 may be used, such as one roller, two rollers, four rollers, etc. In an example where the rotor 1016 includes at least two rollers 1018, as the rotor 1016 rotates, a volume of fluid may become trapped between two of the rollers 1018 to push the fluid along the fluid line 1014. In examples, the pump 1006 may run continuously or may deliver fluid intermittently via partial revolutions. The pump 1006 (e.g., a peristaltic pump), may be low pressure pump. In an instance, the pump 1006 may be capable of delivering pressures of up to 100 PSI and, in some examples, may deliver pressures between 30-70 PSI.

The valve 1010 may also be positioned outside of the wetted path of the fluid delivery system 1000. The valve 1010 may adjust a cross-sectional area of the fluid line 1014 to allow, reduce, restrict, and/or prevent flow of the fluid through the fluid line 1014. For example, the valve 1010 may adjust the cross-sectional area of the fluid line 1014 by applying a force to an exterior of the fluid line 1014. In an instance, the valve 1010 may be a pinch valve that clamps or pinches the fluid line 1014 along an exterior surface of the fluid line 1014. Similar to the pump 1006, the fluid line 1014 may be removable from the valve 1010 and replaceable with the same or different fluid line 1014 (e.g., when a fluid line is contaminated, old, or otherwise desired to be replaced, or if the valve 1010 is moved to a different fluid delivery system 1000). Thus, the valve 1010 may be reusable (e.g., without cleaning, sanitation, or sterilization).

Although the pump 1006 and/or the valve 1010 may be positioned outside of the wetted path, as described above, the fluid delivery system 1000 may instead include a pump 1006 and/or valve 1010 that is along the wetted path. For example, the fluid delivery system 1000 may use a centrifugal pump, a high-pressure pump, etc. In other examples, the fluid delivery system 1000 may include a valve 1010 in the wetted path, such as a pressure membrane, a check valve, or other valve 1010 in fluidic contact with the fluid in the fluid line 1014.

The fluid reservoir 1004 may be any container for storing a fluid (e.g., water or medicine). For example, the fluid reservoir 1004 may be a bag, tank, etc. The fluid reservoir 1004 may be refillable. In an example, the fluid reservoir 1004 may include a one-way valve to allow fluid to exit the fluid reservoir 1004 into the fluid line 1014 without backflow or contamination of the fluid in the fluid reservoir 1004. Alternatively, the fluid reservoir 1004 may be disposable and/or replaceable.

The pressure chamber 1008 may be any storage means capable of holding a fluid and/or gas under pressure (e.g., relative to ambient pressure outside of the pressure chamber 1008). For example, the pressure chamber 1008 may be capable of holding a pressure of up to 200 PSI. In an example, the pressure chamber 1008 may be a medical balloon. Release of fluid from the pressure chamber 1008 may be based on a valve 1010 (e.g., as described above) positioned between the pressure chamber 1008 and a nozzle 1012. For example, the valve 1010 may control release of the pressurized fluid from the pressure chamber 1008 for entry into the nozzle 1012 for injection into a flow of breathing gases into the breathing circuit 1002. The pressure chamber 1008, therefore, is configured to store fluid at a pressure at which flow into the nozzle 1012 causes aerosolization and/or atomization without dripping (e.g., a minimum pressure of the fluid at which the nozzle 1012 safely/cleanly injects fluid into the flow of breathing gases). To stabilize pressure of the fluid in the pressure chamber 1008, the pressurized chamber 1008 may include a compressible volume in addition to a fluid volume (e.g., a volume of the fluid stored in the pressure chamber, which may be incompressible). For example, the compressible volume may be a volume of air. Alternatively, the compressible volume may be foam or other compressible material to build pressure in the pressure chamber 1008 as the pressure chamber 1008 fills (e.g., a force is applied by the compressible volume as the fluid compresses the compressible material as the fluid volume expands).

The pressure chamber 1008 is positioned between the pump 1006 and the nozzle 1012. By using a pressure chamber 1008, a response time and/or pressure generating profile of the pump 1006 may be decoupled from the injection of fluid. For example, if a pump 1006 has a slow response time or includes a ramp-up period before obtaining a minimum pressure of the fluid, then the pressure chamber 1008 may be charged by the pump for controlled dispersion by the valve 1010, rather than controlled dispersion based on the response time of the pump 1006. In an alternative example described below with respect to FIGS. 11A-11B, a pressure chamber 1008 and valve 1010 may be removed from the fluid delivery system 1000 when the pump 1006 is capable of a pressure profile that is square and/or spiked (e.g., the pump 1006 is capable of ramping up pressure of the fluid quickly, such as less than one second or less than a half of a second).

The nozzle 1012 may be similar to other nozzles described herein, such as injection tip 500 or nozzles 804, 904, 944. As also described herein, the nozzle 1012 may be integrated into a breathing circuit at one or more points, such as along the breathing circuit 1002 (e.g., along the inspiratory line or after the wye) or at a patient interface (e.g., at an invasive patient interface, such as an endotracheal tube, or at a non-invasive patient interface). The nozzle 1012 may be couplable to the breathing circuit 1002, such that the fluid delivery system 1000 may deliver fluid to the nozzle for injection into the breathing circuit 1002. As further described herein, the nozzle 1012 may be disposable and/or replaceable. Additionally, a minimum pressure of the fluid delivered to the nozzle 1012 (e.g., via the pressure chamber 1008 and valve 1010, or directly from the pump 1006) may be determined based on properties of the nozzle 1012 (e.g., quantity of orifices, orifice size(s), orifice layout, internal diameter of the nozzle 1012, etc.). For example, different properties of the nozzle 1012 may be associated with different fluid resistance of the nozzle 1012 and thus also associated with whether fluid is either aerosolized and/or atomized or drips from the nozzle 1012 into the breathing circuit 1002. The pressure at which the fluid aerosolizes and/or atomizes instead of dripping is the minimum pressure (e.g., driving pressure) to be delivered from the pressurized chamber 1008 or the pump 1006.

The pump 1006 and/or the valve 1010 may be controlled to deliver fluid to the nozzle 1012 for injection into the breathing circuit 1002. In an example, a controller (e.g., such as controllers 110, 714, 814, 914, 938) may control the pump 1006 and/or the valve 1010. In the example fluid delivery system 1000 shown in FIGS. 10A-10B, the pump 1006 and/or the valve 1010 may be controlled based on a pressure in the pressure chamber 1008. For example, control of the pump 1006 and/or valve 1010 may be based on measurements from a pressure sensor in the pressure chamber 1008 (e.g., in the wetted path), force-loading feedback from an external force sensor on the pressure chamber 1008 (e.g., outside of the wetted path), a load exerted on the motor of the pump 1006 due to pressure buildup in the pressure chamber 1008 (e.g., the pump 1006 may automatically turn off when a load on the pump motor/rotor exceeds a threshold load), and/or a volume sensor correlating a volume of fluid inside of the pressure chamber 1008 with a pressure (e.g., an optical volume sensor outside of the wetted path), and/or any other sensor inside or outside of the wetted path to determine a pressure inside of the pressure chamber 1008. In examples, the pump 1006 may be controlled (e.g., turned on or off) to maintain a minimum pressure, maximum pressure, and/or range of pressures inside of the pressure chamber 1008. For instance, the pump 1006 may be controlled to turn on when the pressure in the pressure chamber 1008 drops below a minimum pressure (e.g., 40 PSI, 50 PSI, 80 PSI, 100 PSI, etc.) and turn off when the pressure in the pressure chamber 1008 reaches a maximum pressure (e.g., 150 PSI, 160 PSI, 170 PSI, 180 PSI, 190 PSI, 200 PSI, 250 PSI, etc.). Additionally, the valve 1010 may be controllable to open (partially or fully) or close based on the pressure in the pressure chamber 1008, such as only opening if at least a minimum pressure is measured in the pressure chamber 1008.

One or more components of the fluid delivery system 1000 may be disposable. For example, the fluid line 1014 of the fluid delivery system 1000 may be disposable and/or replaceable. Additionally, the pressure chamber 1008 and/or the nozzle 1012 may be disposable and/or replaceable. The fluid reservoir 1004 may be refillable, disposable, and/or replaceable. In an example, the fluid reservoir 1004, the fluid line 1014, the pressure chamber 1008, and the nozzle 1012 may be disposed and/or replaced. In this example, the pump 1006 and/or the valve 1010 may be reusable (e.g., not contaminated if located outside of the wetted path). Disposability of lower cost components of the fluid delivery system 1000 while reusing more expensive capital equipment (e.g., pumps, valves) may allow for low cost patient care and less time required for cleaning and sanitizing of equipment between patients and/or systems.

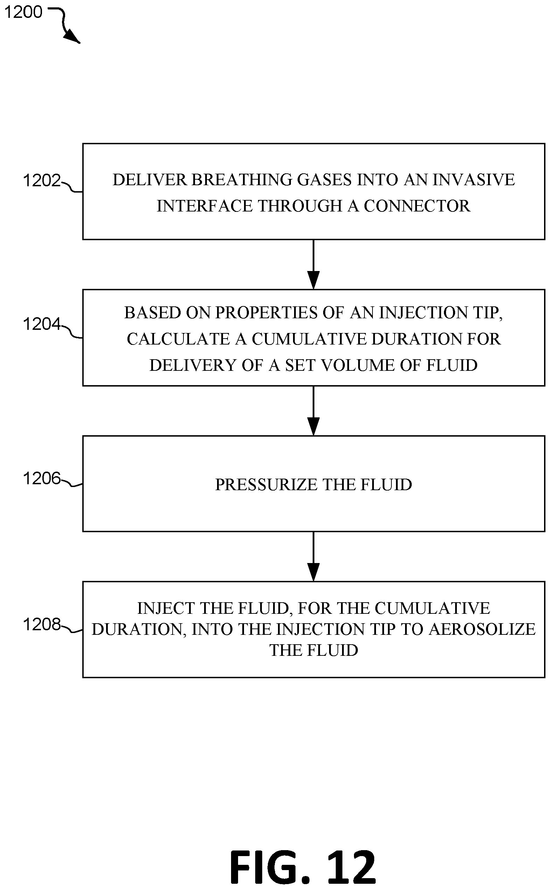

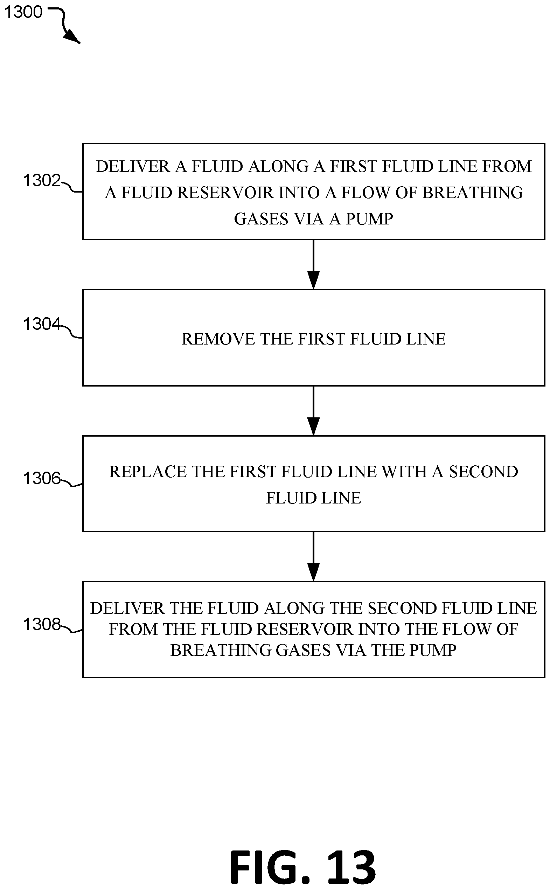

FIGS. 11A-11B show an example fluid delivery system 1100 without a pressure chamber (e.g., pressure chamber 1008) and without a valve (e.g., valve 1010). As shown, the fluid delivery system 1100 includes a fluid reservoir 1104 (which may be similar to fluid reservoir 1004), a fluid line (which may be similar to fluid line 1014), a pump 1106 (which may be similar to pump 1006), and a nozzle 1112 (which may be similar to nozzle 1012). The fluid delivery system 1100 injects fluid into breathing circuit 1102 (similar to breathing circuit 1002) via the nozzle 1112. As described above, a pump 1106 may be directly coupled to a nozzle 1112, as shown in FIGS. 11A-11B, when the pump 1106 is capable of obtaining a minimum pressure within a minimum ramp-up period (e.g., one second, a half of a second, 0.2 seconds, etc.). The minimum ramp-up period is associated based on properties of the nozzle 1112, such that the pressure of fluid delivered to the nozzle 1112 results in aerosolization or atomization without dripping into the breathing circuit 1102. Additionally, in FIGS. 11A-11B, the fluid reservoir 1104, fluid line 1114, and/or nozzle 1112 may be disposable and/or replaceable. The pump 1106 may be outside of the wetted path such that the pump 1106 is reusable. FIGS. 12 and 13 show example methods according to the disclosed technology. The example methods include operations that may be implemented or performed by the systems and devices disclosed herein. For example, at least systems 100, 200, 800, 900, 1000, and 1100 depicted in FIGS. 1, 2, 8, 9A-9D, 10A-10C, and 11A-11B may perform the operations described in the method. In addition, instructions for performing the operations of the methods disclosed herein may be stored in a memory of a ventilator, a fluid delivery system, and/or a humidification system (e.g., memory 112 in FIG. 1, memory 208 in FIG. 2, memory 716 in FIG. 7, memory 816 in FIG. 8, or memories 916, 940 in FIGS. 9A-9D).

FIG. 12 shows a flowchart illustrating a method 1200 for delivering a fluid into an invasive patient interface. At operation 1202, breathing gases are delivered into an invasive interface (e.g., endotracheal tube) through a connector (e.g., connector 300, 402, 702, 802, 902). As described herein, the connector includes multiple connection ports. A primary port of the connector provides an opening to a primary channel of the connector. A secondary port of the connector provides an opening to a secondary channel of the connector. The secondary channel is fluidly coupled to the primary channel. Breathing gases may be provided by a ventilator (e.g., system 102 or ventilators 801, 901) via ventilation tubing of an inhalation limb fluidly coupled to the connector. The breathing gases may flow through the primary channel (e.g., via ventilation tubing coupled to the primary port) and into an invasive interface (e.g., an endotracheal tube) fluidly coupled to the connector.

At operation 1204, based on properties of an injection tip, a cumulative duration for delivery of a set volume of a fluid is calculated. The injection tip (e.g., injection tip 500) may include a body having an inner cavity or channel extending through the body. Additionally, the injection tip may be configured to removably couple to the secondary channel of the connector at the secondary port. The diameter of the inner cavity defines an inner diameter of the injection tip through which a fluid may flow. The inner diameter may vary across a length of the injection tip. The injection tip may also include a membrane configured to span the inner diameter (e.g., positioned cross-flow or across a cross section of the inner diameter). The membrane may include orifices to allow flow of a fluid through the injection tip to proceed through the orifices. There may be a specified quantity of orifices having one or more sizes and positions about the membrane. Thus, in examples, properties of the injection tip may include an inner diameter of the injection tip, a quantity of orifices, and size of the orifices. When a fluid flows through the inner cavity of the injection tip and is pressurized against the membrane having the orifices, the fluid is formed into droplets.

In addition to the properties of the injection tip, fluid properties of the fluid (e.g., viscosity, density, etc.), and/or a pressure of the fluid against the membrane may govern a size of the droplets of fluid and speed at which the droplets form. The size and quantity of the droplets and droplet formation speed (e.g., velocity of the droplets) accumulate in total volume of fluid over a given cumulative duration. The size and/or velocity of the droplets is based on a pressure applied to the fluid against the set of orifices, the configuration of the set of orifices, and a fluid flow property of the fluid. For example, smaller droplets may result from higher pressures, smaller orifices, and higher viscosities. In other examples, the velocity of the droplets may increase with higher pressures, larger orifices, and lower viscosities.

The cumulative duration for delivery of the fluid may be calculated based on a set volume of the fluid to be delivered, properties of the injection tip, fluid properties of the fluid (e.g., viscosity, density, etc.), and/or a pressure of the fluid against the membrane. The set volume of a fluid may be a prescribed volumetric dosage for a given patient. The set volume may be identifiable or set by a clinician, ventilator, and/or fluid delivery system based on patient information (e.g., body weight, predicted body weight, gender, other medications being delivered, past history with the fluid, etc.). In an example, the fluid is a medicine or drug with known fluid flow properties (e.g., viscosity, density, etc.). The pressure of the fluid against the membrane, as further described below in operation 1206, may be constant or variable.

The cumulative duration calculated for the set volume of fluid may be delivered phasically. For example, a breathing phase (e.g., inhalation phase or exhalation phase) may be determined and the cumulative duration may be selectively delivered during one type of breathing phase. In an instance, the cumulative duration spans only inhalation phase(s). The cumulative duration may include multiple dose periods, which may span one or more breaths. For example, if the cumulative duration is greater than a duration of an inhalation phase, then the cumulative duration may be spaced and segmented across the inhalation phase of multiple breaths (e.g., multiple dose periods). Additionally, the cumulative duration may be further restricted to specific portions of a phase. For example, the cumulative duration may be applied during a first half of an inhalation phase, a first third of an inhalation phase, or a first quarter of an inhalation phase. When droplets of a fluid of a known size and quantity are delivered for the calculated cumulative duration, the total volume of fluid delivered accumulates in the set volume of the fluid.

At operation 1206, the fluid is pressurized. The fluid may be pressurized manually (e.g., via a syringe) or mechanically (e.g., via a pump). In an example where the fluid is pressurized mechanically, a valve may be positioned between the injection tip and the pressure actuator. The valve may control when and for how long the pressurized fluid is injected into the injection tip, as further described below in operation 1208. The valve may be a solenoid valve. The valve may be opened and closed according to a pulse width modulation.

At operation 1208, the fluid is injected, for the cumulative duration, into the injection tip to aerosolize the fluid. As described above, when a pressurized fluid is provided into an injection tip, the fluid is formed into droplets to cause aerosolization of the fluid. As also described above, the injection tip may be coupled to a secondary port of the connector described in operation 1202. When the injection tip is coupled to the secondary port, the aerosolized fluid travels through the secondary channel of the connector. As the aerosolized droplets of fluid reach the end of the secondary channel, the droplets are aerosolized and suspended in the breathing gases flowing through the primary channel. The breathing gases in the primary channel then carry the aerosolized fluid out of the connector and into the invasive interface for delivery into the patient's airway and lungs, for absorption by the patient. Thus, the aerosolized fluid travels through the secondary channel and is carried into the invasive interface by the breathing gases in the primary channel.

FIG. 13 shows a flowchart illustrating a method 1300 for delivering a fluid into a breathing circuit. At operation 1302 a fluid is delivered along a first fluid line (e.g., fluid lines 1014, 1114) from a fluid reservoir (e.g., fluid reservoir 1004, 1104) into a flow of breathing gases via a pump (e.g., pump 1006, 1106). The pump is positioned between the fluid reservoir and the flow of breathing gases. In an example, a nozzle (e.g., nozzle 804, 904, 944, 1012, 1112, or injection tip 500) is coupled to a breathing circuit (e.g., inhalation limb, wye, between the wye and the patient interface) or a patient interface (e.g., invasive or noninvasive interface) through which the breathing gases are flowing to a patient. When the fluid delivered to the nozzle is at least a minimum pressure, the fluid is aerosolized and/or atomized through the nozzle, without dripping, into the flow of breathing gases.

A pressure chamber (e.g., pressure chamber 1008) and a valve (e.g., valve 1010) may be positioned between the pump and the nozzle. The pressure chamber may maintain fluid at a minimum pressure, maximum pressure, or range or pressures. The valve may control flow of the fluid from the pressure chamber to the nozzle. The pump and/or valve may be controlled based on a pressure measured or estimated of the fluid in the pressure chamber. The pressure in the pressure chamber may be estimated using a pressure sensor, external force sensor, volume sensor, load sensor, etc.

As also described herein, the pump, valve, and/or fluid reservoir may be reusable. For example, the pump and/or valve may be positioned outside of the wetted path of the fluid delivery system. By being positioned outside of the wetted path, components are not in fluidic contact with fluid being delivered through the nozzle and thus not contaminated by the fluid and not a risk to introduce contaminants to the fluid. The fluid reservoir may include a one-way valve or check valve to prevent backflow of fluid into the fluid reservoir and otherwise prevent contamination of the fluid reservoir. The fluid reservoir may thus be reused and/or refilled. Alternatively, the fluid reservoir may be disposable and/or replaceable. Other components of the fluid delivery system may be disposable, such as the first fluid line, the pressure chamber, and/or the nozzle.

At operation 1304, the first fluid line is removed. At operation 1306, the first fluid line is replaced with a second fluid line. As described herein, a fluid line of the fluid delivery system may be disposable and/or replaceable. Other components may be reusable, such as a pump and/or valve. Replacement of the first fluid line with the second fluid line may include removing the first fluid line from the pump and/or valve (as applicable) and/or other reusable components and feeding the second fluid line through the pump and/or valve (as applicable) and/or other reusable components of the fluid delivery system. In examples, the nozzle is also replaced with a new nozzle. In further examples, a pressure chamber and/or fluid reservoir are also replaced. Replacement of one or more components may include disposal of the original components being replaced. For example, the first fluid line may be disposed of after being replaced by the second fluid line. Removal and/or replacement of any components of the fluid delivery system may be performed when delivery of a fluid has stopped or paused, and prior to resuming delivery of the fluid via the fluid delivery system.

At operation 1308, the fluid is delivered along the second fluid line from the fluid reservoir into the flow of breathing gases via the pump. As described herein, the fluid is delivered from the fluid reservoir (or replacement fluid reservoir holding the same or different fluid as the fluid run through the first fluid line at operation 1302) for injection into a flow of breathing gases via the pump (which is reused). The fluid may be injected into the flow of breathing gases at a nozzle (e.g., the same nozzle as described at operation 1302 or a replacement nozzle). The nozzle may be provided fluid directly from the pump, or instead may be provided fluid via a valve releasing fluid from a pressure chamber supplied by the pump.

Reuse and replacement of various components of the fluid delivery system may recur. For example, the second fluid line may be replaced by a third fluid line, etc. A first nozzle may be replaced by a second nozzle, which may be replaced by a third nozzle, etc. Replacement of one or more components of the fluid delivery system (e.g., fluid line(s), fluid reservoir, pressure chamber, and/or nozzle, etc.) may occur simultaneously (e.g., after delivery of the fluid has stopped and prior to re-delivery of the fluid using replaced components of the fluid delivery system). Alternatively, replacement of the component(s) may be independent of each other and/or at different times (e.g., consecutive and/or not simultaneous).

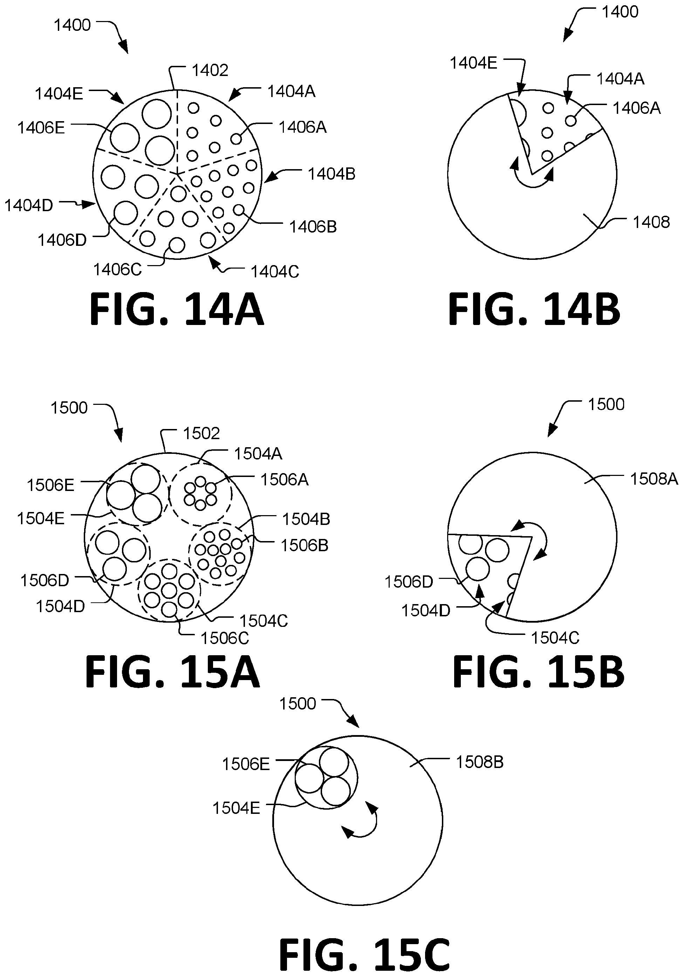

FIGS. 14A-14B, 15A-15B, 16A-16C, 17A-17B, 18A-18E, and 19A-19D show additional configurations of various components described above. For example, different configurations are described for at least the following components: injection tips, orifices on injection tips, and connectors. Different configurations of the various components may allow for enhanced control over droplet size for dispersion into breathing gases of a patient. The various configurations may account for a range of different properties of fluid being aerosolized (e.g., viscosity, density, etc.) such that the fluid may not need to be diluted to specific viscosities for aerosolization. Significant dilution of a drug being delivered to a patient may contribute to errors in dosage and absorption by the patient.

FIGS. 14A-14B show an additional configuration 1400 for sets of orifices on a membrane 1402 of an injection tip. FIGS. 15A-15B show another configuration for sets of orifices of an injection tip. The sets of orifices and the membranes 1402, 1502 may have similar properties or characteristics to those described above with respect to FIGS. 6A-6C. In the examples shown in FIGS. 14A-14B and 15A-15B, the membrane is circular. A membrane 1402 of any shape, however, is appreciated.

Unlike FIGS. 6A-6C, in FIGS. 14A-14B and 15A-15B, there are a plurality of sets of orifices on each membrane 1402, 1502. For example, the membrane 1402 shown in FIGS. 14A-14B is divided into sectors, with each sector having a set of orifices (e.g., a first sector 1404A has a first set of orifices 1406A, a second sector 1404B has a second set of orifices 1406B, a third sector 1404C has a third set of orifices 1406C, a fourth sector 1404D has a fourth set of orifices 1406D, and a fifth sector 1404E has a fifth set of orifices 1406E). Each of the sectors may be defined by an arc length along a circumference of the membrane 1402 and the two radii connecting the center of the membrane 1402 with each end of the arc. Each sector may have the same area as any other sector. In the example shown in FIGS. 14A-14B, the membrane 1402 is subdivided into five, equal sectors. Although five sectors are shown, any number of sectors is appreciated (e.g., two, three, four, six or more sectors). The orifices of the sets of orifices on the membrane 1402 may be evenly or unevenly distributed about the sectors in which they are contained. Each set of orifices may include orifices of uniform size or orifices of varying sizes (e.g., as shown in FIG. 6B).

A quantity of orifices in each set of orifices may depend on the size of the sector and/or the size of the orifices. For example, in sectors of equal size (e.g., as shown in FIGS. 14A-14B), the bigger the orifices, the less orifices that are capable of fitting into the sector. In an instance, a sector (e.g., first sector 1404A, second sector 1404B, third sector 1404C) may include at least four orifices (e.g., in each respective set of orifices 1406A, 1406B, 1406C) and another sector having larger orifices (e.g., fourth sector 1404D, fifth sector 1404E) may include four or less orifices (e.g., in each respective set of orifices 1406D, 1406E).

In some instances, sectors may include more or less orifices regardless of orifice size. For example, if two sectors have a common, uniform orifice size, each sector may have a different quantity of orifices. For example, as shown in FIGS. 14A-14B, a first sector 1404A and a second sector 1404B have a common, uniform size for each orifice 1406A, 1406B, however, the first sector 1404A includes less orifices 1406A than the second sector 1404B.

The sectors 1404A-1404E may be organized about the membrane 1402. If the membrane 1402 is an ellipse (e.g., in an instance the ellipse may be a circle, as shown), the sectors 1404A-1404E may be organized or distributed radially. Organization or ordering may be based on quantity of orifices in a set of orifices, sizes of orifices, total area of the set of orifices, a viscosity of liquid accommodatable through the set of orifices, maximum flow of fluid through a set of orifices, droplet size through a set of orifices, pressure or force needed to aerosolize a fluid through the set of orifices, a type of drug or drugs capable of being aerosolized through the set or orifices, etc. For instance, sectors may be organized in a clockwise or counterclockwise pattern based on a popularity, or frequency of use of drug(s) associated with a set or orifices. A first drug (or first viscosity), with a highest frequency of use, may be associated with the set of orifices 1406A included in a first sector 1404A. A second sector 1404B, positioned adjacent, clockwise of the first sector 1404A, may have a second set of orifices 1406B that is associated with a second drug (or second viscosity) having a next highest frequency of use. In another instance, sectors may be organized clockwise or counterclockwise from lowest flow (e.g., at a specific pressure) and highest flow (e.g., at the same, specific pressure).

FIG. 14B shows the membrane 1402 of FIG. 14A with an orifice-selectable shutoff 1408 (e.g., cover 1408). In the example shown in FIG. 14B, the shutoff 1408 is rotatable about the membrane 1402 to allow flow through one of the sets of orifices on the membrane 1402. The shutoff may have an opening that is a shape of at least one of the sectors of the membrane 1402. The set of orifices exposed by the opening of the shutoff (e.g., the set of orifices through which flow is unrestricted and not shut off) are selected for flow of liquid through the membrane 1402. For example, when the shutoff 1408 is rotated such that the opening (e.g., an opening the size of sectors 1404A-1404E, which in the example shown in FIG. 14A are of same size and shape) exposes at least one of the sets of orifices (e.g., overlaps with one of the sectors), the sector exposed by the opening is "selected" so that flow through the membrane 1402 at that set of orifices is exposed while flow through any other orifices is restricted or prevented (e.g., flow of fluid is shut off or blocked from flowing through any other orifices covered by the shutoff 1408). Rotation of the shutoff 1408 is controllable. Control of the selected set of orifices may be manual (e.g., via manual rotation of the shutoff) or automatic, based on a selection of one of the organizational features of the sets of orifices, described above. For example, a clinician may select a drug to be delivered via a set of orifices and the shutoff may be rotated (e.g., automatically or by providing an instruction to a clinician for an amount of rotation) to open an associated, desired set of orifices.

As another example, the membrane 1502 shown in FIGS. 15A-15C includes portions each having a set of orifices (e.g., a first portion 1504A has a first set of orifices 1506A, a second portion 1504B has a second set of orifices 1506B, a third portion 1504C has a third set of orifices 1506C, a fourth portion 1504D has a fourth set of orifices 1506D, and a fifth portion 1504E has a fifth set of orifices 1506E). Each portion may be a same or different shape than the other portions. The portions may be non-overlapping. In the example shown in FIGS. 15A-15C, the portions are elliptical (e.g., circular). Although five potions are shown, any number of portions is appreciated (e.g., two, three, four, six or more portions). The orifices of the sets of orifices on the membrane 1502 may be evenly or unevenly distributed about the portions in which they are contained. Each set of orifices may include orifices of uniform size or orifices of varying sizes. Similar to that described for FIGS. 14A-14B, a quantity of orifices in each portion (e.g., in each set of orifices) may depends on size(s) of the orifices and/or size(s) of the portions. Additionally, the portions may be organized about the membrane 1502.

FIGS. 15B-15C shows the membrane 1502 of FIG. 15A with an orifice-selectable shutoff 1508A, 1508B (e.g., cover 1508A, 1508B). The shutoff 1508A of FIG. 15B is similar to that described with respect to FIG. 14B. For example, the shutoff 1508A may have an opening that is a sector. The sector opening may be sized and shaped to overlap with at least one of the portions having a set of orifices. The opening of the shutoff 1508B may be a different shape to better accommodate the portions of the membrane 1502. For example, the opening of the shutoff 1508B may be the same shape as a portion of the membrane 1502. For instance, each portion 1504A-E may be an ellipse of a same size and the opening may be a matching ellipse. The opening may then be rotated or moved about the membrane 1502 to selectively allow flow of fluid through a set of orifices in at least one portion of the membrane 1502.

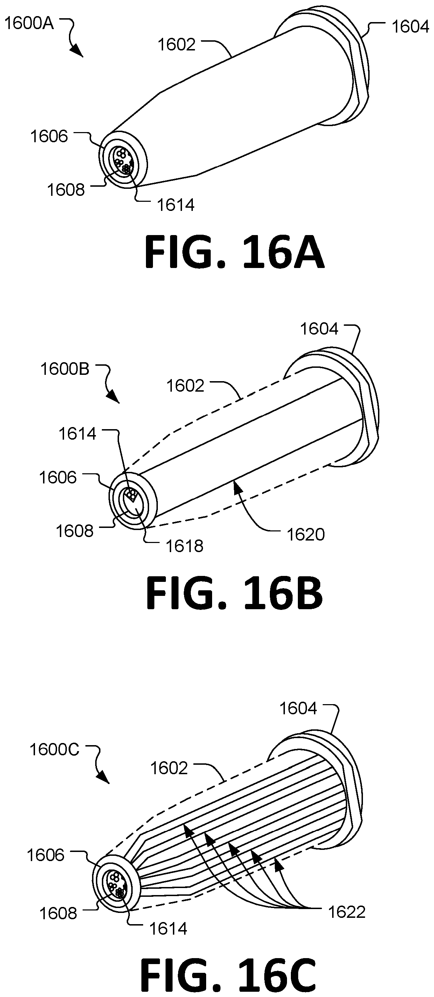

Although the selection mechanisms described in FIGS. 14B, 15B, and 15C refer to a shutoff that is rotatable through different sets of orifices on a membrane, other selection mechanisms for flow through one set of orifices is appreciated. For example, the injection tip and/or membrane may be rotatable to cycle through the sets of orifices. For instance, an injection tip may have a fluid delivery line couplable to at least a portion of the membrane (e.g., such as that described at least with respect to FIG. 16B). The tip and/or membrane may be moved or rotated to align at least one set of orifices with the fluid delivery line. In another example, an entire tip or membrane may be changeable. In a further example, stylets with specific nozzle tips may be fed through a lumen into the breathing circuit, into a connector, and/or into an injection tip. Stylets may be color-coded to indicate different nozzle tips.

FIGS. 16A-16C show other configurations of an injection tip. An example of the shutoff 1618 on an injection tip 1600B is shown at least with respect to FIG. 16B. In an instance, an injection tip 1600B having a shutoff 1618 may include one fluid delivery line 1620 that feeds fluid to all sets of orifices 1614 on the membrane 1608. The fluid delivery line 1620 may be contained in a body 1602 of the injection tip 1600B and fluidly couple a first end 1604 of the body 1602 with a second end 1606 of the body 1602 (e.g., a second end 1606 of the body 1602 having the membrane 1608). Alternatively, the shutoff 1618 may be removable to expose all sets of orifices 1614 on the injection tip 1600A, 1600C, such as that shown in FIGS. 16A and 16C.

In an example where the membrane 1608 does not have a coupled shutoff 1618, each set of orifices 1614 may be fed via separate fluid delivery lines 1622. Each fluid delivery line 1622 may be independently controllable to selectively feed one or more sets of orifices at a time. In the example shown in FIG. 16C, five sets of orifices 1614 are each coupled to an independent fluid delivery line 1622 (e.g., five fluid delivery lines 1622). The fluid delivery lines 1622 may be contained in the body 1602 of the injection tip 1600C and fluidly couple a first end 1604 of the body 1602 with a second end 1606 of the body 1602 (e.g., a second end 1606 of the body 1602 having the membrane 1608). In an instance where the sets of orifices are grouped in sectors of different sizes, the fluid delivery lines 1622 may have different (e.g., proportional) internal diameters to allow more or less volumetric flow of fluid. Alternatively, the fluid delivery lines 1622 may all have the same internal diameter.

The membrane 1608, orifices 1614, second end 1606, body 1602, fluid delivery lines 1620, 1622, or any other component, internal or external, of the injection tip 1600A-C may include an anti-microbial coating and/or an anti-microbial material (e.g., silver alloy) to minimize build-up or clogs of the injection tip (e.g., from humidity, fluids from the patient, etc.). The coating and/or material may be hydrophobic and/or oleophobic.

The injection tip 1600A-C may have specified geometrical features. For example, the tip of the nozzle at the second end 1606 (e.g., the membrane end) may be 1-5 mm in diameter (e.g., 2 mm, 2.5 mm, 3 mm, etc.). An orifice 1614 may be 0.1-1.0 mm in diameter (e.g., 0.3, 0.4, 0.5, etc.). The injection tip 1600A-C may have a defined length to depth (L/D) ratio, such as 2:1, 3:1, 4:1, etc. In an instance, the length of the injection tip 1600A-C is 1.2 mm and the diameter of the orifice(s) are 0.4 mm.

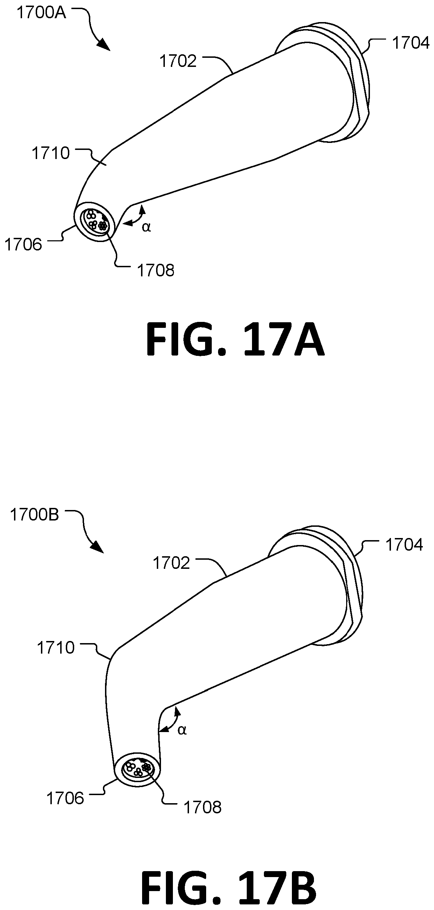

FIGS. 17A-17B show other configurations of an injection tip 1700A-B, in addition to those described with respect to FIGS. 5, 16A-C. In particular, FIGS. 17A-17B show injection tips 1700A-B with a bend 1710 of angle α along the body 1702 between the first end 1704 and the second end 1706. The bend 1710 may direct flow of aerosolized fluid through orifices perpendicular to the membrane 1708, at an angle α relative to the first side 1704 of the body 1702.

An angle α of the bend 1710 may be based on a direction of air flow relative to the membrane 1708 when the injection tip 1700A-B is inserted for injection of fluid into a breathing circuit. By focusing the dispersion of fluid droplets in the same direction as airflow and away from sidewalls of the breathing circuit, rainout along a sidewall is reduced and absorption of the fluid by the patient is improved. The fluid droplets may be biased by air flow around the dispersion (e.g., airflow near the membrane 1708). By aligning the dispersion of fluid droplets relatively in line with air flow (e.g., via a bend 1710 in the injection tip 1700A-B), the fluid droplets may be better carried downstream in the breathing circuit while reducing bias flow of fluid droplets into a sidewall of the breathing circuit. In some examples, the bend 1710 of the injection tip 1700A-B may align the membrane 1708 with a middle of a flow of breathing gases (e.g., when the injection tip is inserted into the breathing circuit) to disperse fluid droplets directly downstream with the air flow. In this example, the membrane 1708, during injection, is axially aligned with the flow of breathing gases in the breathing circuit. In an example, an angle α of the bend 1710 is between 15-180 degrees, such as 30-90 degrees, 45-90 degrees, 45 degrees (e.g., as shown in the injection tip 1700A in FIG. 17A), 90 degrees (e.g., as shown in the injection tip 1700B in FIG. 17B), etc.

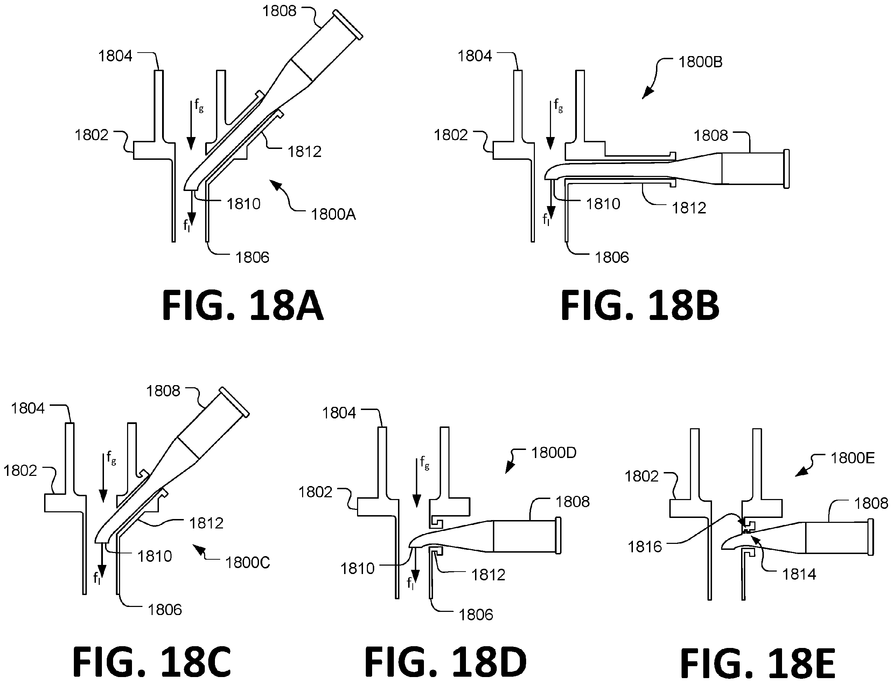

FIGS. 18A-18E show different configurations 1800A-E of a connector 1802 for coupling ventilation tubing to an endotracheal tube and an injection tip 1808 insertable into the connector 1802. In each of the configurations 1800A-E shown in FIGS. 18A-18E, a bend in the injection tip 1808 may be desirable to direct a flow of dispersed fluid droplets fₗ inline with a flow of breathing gases f_{g}. The geometries of the connector 1802 and the injection tip 1808 may be designed to maintain appropriate air flow of breathing gases delivered to the patient (e.g., minimal interference of the injection tip 1808 with the flow of breathing gases f_{g}, which flow from a gas inlet end 1804 of the connector 1802 to an outlet 1806). Additionally, the geometry of the injection tip 1808, as further described with respect to FIGS. 17A-17B may be designed to streamline and focus the dispersion and flow of dispersed fluid droplets fₗ in a middle of the flow of breathing gases f_{g}.

As shown in FIGS. 18A, 18C an injection end 1810 of the injection tip 1808 may be bent 10-80 degrees to align the flow of dispersed fluid droplets fₗ with the flow of breathing gases f_{g}. Alternatively, in FIGS. 18B, 18D, and 18E, an injection end 1810 of the injection tip 1808 may be bent approximately 90 degrees to align the flow of dispersed fluid droplets fₗ with the flow of breathing gases f_{g}. The bend may be based on an angle between the tip insertion line 1812 and the flow of breathing gases f_{g}. Additionally, a length of the injection tip 1808 may be based on a length of the tip insertion line 1812. For example, the tip insertion line 1812 and the injection tip 1808 are shorter in FIGS. 18C-18E than in FIGS. 18A-18B.

The bend in the injection tip 1808 may be designed to substantially remain inline with the length of the injection tip 1808 to allow insertion and removal of the injection tip 1808 via the tip insertion line 1812 of the connector 1800A-E. Retaining the length of the injection tip 1808 along the length may be particularly important with greater lengths of the tip insertion line 1812 because there is less error to move or angle the injection tip 1808 during insertion and removal. Additionally, as shown in FIG. 18E, to ensure correct orientation of the bend during insertion of the injection tip 1808, the injection tip 1808 and/or the connector 1802 may be keyed. In an example, the injection tip 1808 may include a key 1814 (or an indicator 1814 of orientation). The key 1814 may align with a key 1816 of the connector 1802 or tip insertion line 1812. In an instance the keys may be an orientation-related threaded coupling, an indentation, matching tabs, a visual indicator, a snap-fit, etc.

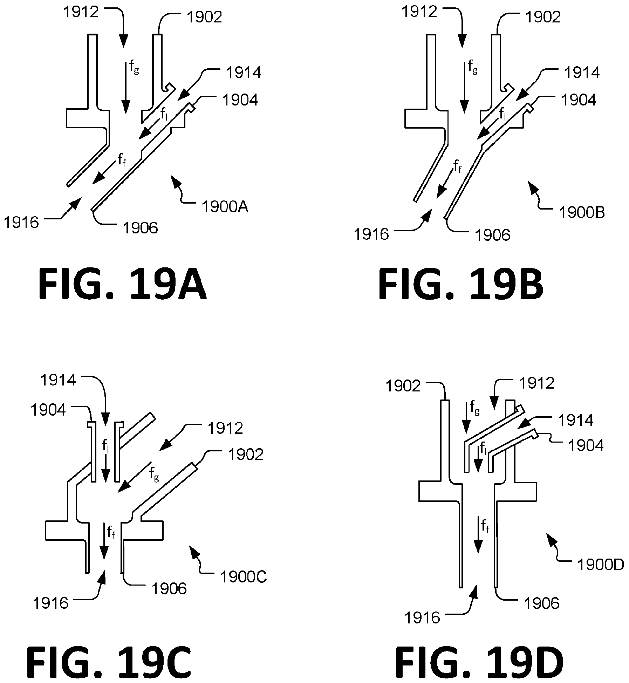

FIGS. 19A-19D show different configurations of a connectors 1900A-1900D for coupling ventilation tubing to an endotracheal tube. Unlike FIGS. 18A-18E, which rely on a bend in the injection tip to align a flow of dispersed fluid droplets fₗ with a flow of breathing gases f_{g} and/or a flow of outlet gases f_{f}, the geometries of the connectors 1900A-1900D shown in FIGS. 19A-19D are shaped to direct the flow of dispersed fluid droplets fₗ, without relying on a bend in an injection tip.

The connectors 1900A-1900D of FIGS. 19A-19D include a gas inlet end 1902 with gas inlet 1912, a fluid inlet end 1904 with a fluid inlet 1914, and an outlet end 1906 with an outlet 1916. A flow of breathing gases f_{g} flows from the gas inlet 1912 towards the outlet 1916. A flow of dispersed fluid droplets fₗ flows into the flow of breathing gases f_{g}. The flow of breathing gases fg and the flow of dispersed fluid droplets fₗ combines to form a flow of outlet gases f_{f} (e.g., a flow of breathing gases carrying the dispersed fluid droplets) that flows towards the outlet 1916 to be delivered to a patient downstream.

In the connectors 1900A, 1900C, 1900D shown in FIGS. 19A, 19C, and 19D, the flow of dispersed fluid droplets fₗ is axially aligned with the flow of outlet gases f_{f}. Alternatively, as shown in FIG. 19B, which shows an adjustment to the connector 1900A shown in FIG. 19A, a connector 1900B may include a flow of outlet gases f_{f} that combines the flow vectors of the flow of breathing gases f_{g} and the flow of dispersed fluid droplets fₗ, such that neither the flow of breathing gases f_{g} or the flow of dispersed fluid droplets fₗ are axially aligned with the outlet flow f_{f}. In this connector 1900B, the flow of outlet gases f_{f} may be adjusted to account for a bias in flow of the dispersed fluid droplets fₗ caused by the flow of breathing gases f_{g}. (e.g., to reduce or prevent impact of the fluid droplets with a sidewall of the connector 1900B when combining with the flow of breathing gases f_{g}).

The connectors 1900C-D shown in FIGS. 19C-19D show a combination of the flows f_{g}, fₗ upstream in the connector 1902 compared to the configuration 1900A shown in FIG. 19A (e.g., a combination downstream in the connector 1900C, 1900D). In FIG. 19A, the outlet 1916 is bent to align with the flow of dispersed fluid droplets fₗ. In FIG. 19C, the gas inlet 1912 is bent relative to the fluid inlet 1914 and the outlet 1916, to allow for an axially-aligned injection of fluid droplets. In FIG. 19D, the fluid inlet 1914 is bent relative to gas inlet 1912 and the outlet 1916, to allow for an axially-aligned injection of fluid droplets.

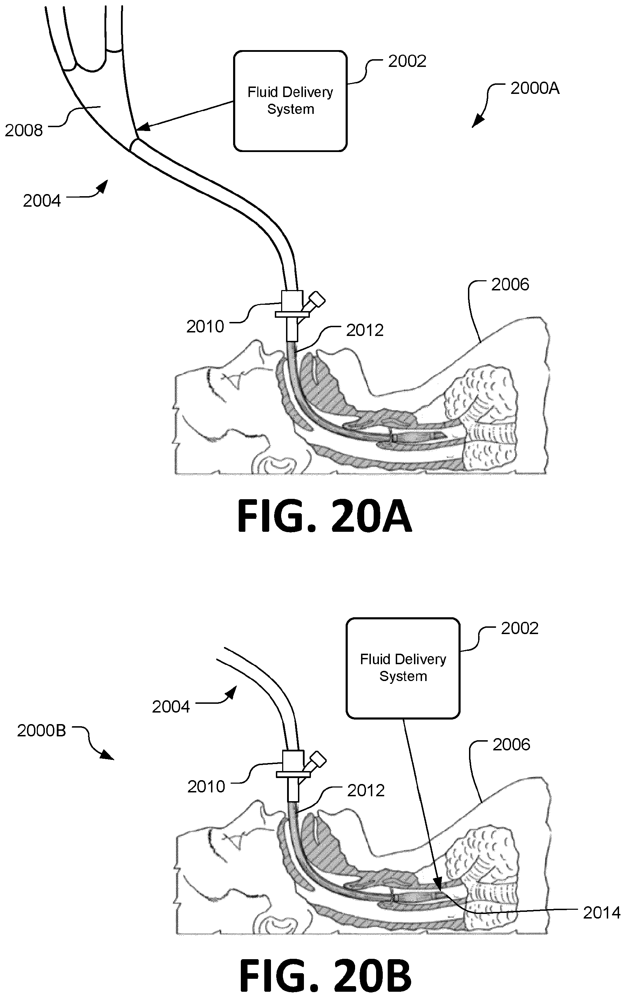

FIGS. 20A-20B are not presently claimed and are maintained for illustration, showing a ventilation system 2000A, 2000B including a fluid delivery system 2002. The ventilation system 2000A, 2000B includes a breathing circuit 2004 including a wye component 2008, a connector 2010 to connect the breathing circuit 2004 with an endotracheal tube 2012, the endotracheal tube 2012 having an outlet end 2014 in an intubated patient 2006.

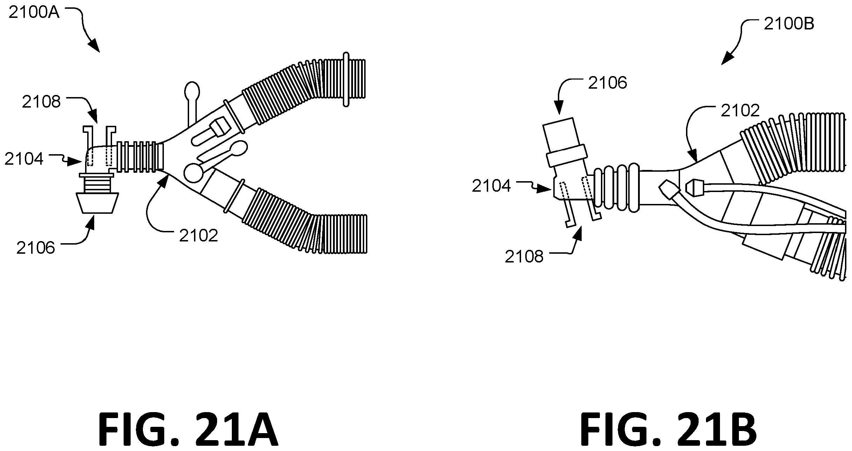

As shown in the ventilation system 2000A of FIG. 20A, the fluid delivery system 2002 may be coupled at a patient side (e.g., downstream side) of the wye component 2008. This configuration is a specific implementation of the ventilation system 900 of FIG. 9C, further described above. Placement of a nozzle downstream of the wye component 2008 to disperse fluid droplets may reduce waste and/or rainout of the fluid droplets by being near the patient 2006 and by limiting interactions with bias flows and filters. Couplings of the fluid delivery system 2002 with the patient side of the wye component 2008 are further described with respect to FIGS. 21A-21B.

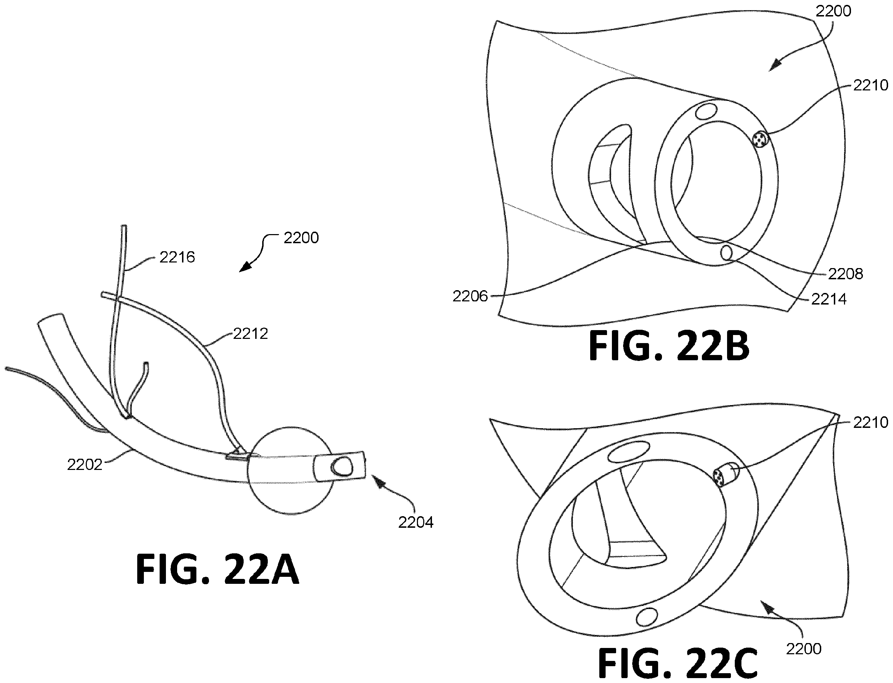

Alternatively, as shown in the ventilation system 2000B of FIG. 20B, the fluid delivery system 2002 may cause delivery of the dispersed fluid droplets at the outlet end 2014 of the endotracheal tube 2012. This ventilation system 2000B may result in direct delivery of dispersed fluid droplets into bronchi and/or lungs of the patient 2006. Couplings of the fluid delivery system 2002 with the outlet end 2014 of the endotracheal tube 2012 are further described with respect to FIGS. 22A-22C.

FIGS. 21A-21B show different configurations of a wye component 2100A, 2100B of a ventilation system (e.g., ventilation system 100, 2000A, etc.). The wye components 2100A, 2100B shown in FIGS. 21A-21B include a junction 2102 where two limbs of a patient circuit combine. Downstream of the junction 2102 (e.g., closer to the patient), the wye component 2100A, 2100B may have a bend 2104 to direct a flow of breathing gases towards an outlet 2106. Near the bend 2104, a fluid droplet inlet 2108 may be oriented to be axially aligned with the outlet 2106. The fluid droplet inlet 2108 may accommodate an injection tip (e.g., as further described herein) and/or a nozzle (e.g., such as a nozzle controllable by a pump, as further described herein). The wye component 2100A, 2100B may be manufactured with the fluid droplet inlet 2108 or, alternatively, may be retrofitted with a fluid droplet inlet 2108.

FIGS. 22A-22C show an endotracheal tube 2200 with an integrated injection tip 2210. The endotracheal tube 2200 includes a breathing gas line 2202 to deliver breathing gases to the patient at an outlet end 2204. The injection tip 2210 may have features similar to that of other injection tips described herein. The breathing gas line 2202 of the endotracheal tube 2200 includes an outer diameter 2206 and an inner diameter 2208. One or more lines (e.g., fluid lines, which may carry liquid and/or gas) may be integrated into the endotracheal tube 2200 between the outer diameter 2206 and the inner diameter 2208. For example, at least one injection tip 2210 may be integrated into the endotracheal tube 2200 and fed by a fluid line 2212. The size of the injection tip 2210 may be less than or equal to a thickness of the wall of the endotracheal tube 2200.

Alternatively, one or more lines may be positioned inside of the inner diameter 2208 of the endotracheal tube 2200 (e.g., lines internal to the endotracheal tube 2200). Internal lines (not shown) may be free floating in the endotracheal tube 2200 or may be spatially held at a specified distance and/or position off of the inner diameter 2208 of the endotracheal tube 2200. Spatially held or positioned internal lines may be angled to disperse fluid droplets and/or gas away from walls of the endotracheal tube 2200 or in a specific direction of a bronchi or lung of a patient.

Lines either internal to or integrated into the endotracheal tube 2200 may be permanent fixtures of the endotracheal tube 2200 or may be removable. In an example, a stylet may be guided through a lumen in the wall of the endotracheal tube 2200 (e.g., between the outer diameter 2206 and the inner diameter 2208) with an outlet at the outlet end 2204 of the endotracheal tube 2200. The wall of the endotracheal tube 2200 may include multiple lumens. In another example, a stylet may be guided through the inner diameter 2208 of the endotracheal tube 2200. The stylet may have an injection tip for delivery of dispersed fluid droplets. The stylet may be guided through the endotracheal tube 2200 via a coupling component, such as at a connector described herein. The stylet may be guided via one or more imaging modalities. In an example, at least a portion of the stylet may be radiopaque.

Additionally or alternatively to angling an injection tip, a gas line may provide a stream of gas to bias the flow of fluid droplets from a nearby injection tip. For example, a gas delivery outlet 2214 may be integrated into the endotracheal tube 2200 and/or contained inside of the endotracheal tube 2200. A gas delivery outlet may direct a stream of gas in a direction of a bronchi or lung. The gas delivery outlet may be a single orifice directing a stream of gas, or may include a set of orifices, similar to injection tips described herein. The gas delivery outlet 2214 may be fed by a gas line 2216 (e.g., which may be partially enclosed in the wall of the endotracheal tube 2200 as a lumen). The stream of gas flowing through the gas delivery outlet may bias a flow of nearby fluid droplets in the direction of the stream of gas (e.g., towards a bronchi or lung of a patient. Any number of injection tips and gas delivery outlets are appreciated (e.g., either integrated into the endotracheal tube 2200 or contained inside of the endotracheal tube 2200). In an instance, the injection tip 2210 is positionable in a first lumen in the wall of the endotracheal tube and a flow of gas is directable through a second lumen to bias the flow of aerosolized liquid from the injection tip 2210. A third lumen may include another injection tip 2210 or may direct another stream of gas. In another instance, a gas delivery outlet may be on a first side of the wall to deliver a flow of gases that may effectively deflect injected droplets to align with the flow of gas. In another example, a flow of gas through a gas delivery outlet may be timed with delivery of fluid droplets. The flow of gas and the delivery of fluid droplets may be in a same or similar direction to reduce or prevent the droplets from hitting the wall of the endotracheal tube and/or an esophageal wall of the patient.

Although only one injection tip 2210 is shown in FIGS. 22A-22C, multiple injection tips may be integrated into, or positioned inside of or relative to, a single endotracheal tube 2200. In an example, a first injection tip may target a first bronchi of a patient (e.g., a right bronchi/lung or a left bronchi/lung) and a second injection tip may target a second bronchi of the patient. Targeting may be accomplished via bias flow from gases near each injection tip or from angling of each injection tip. Targeting delivery of fluid droplets to a specific lung of a patient may be beneficial in surgery or as a surfactant for a collapsed lung. In an endotracheal tube 2200 with multiple injection tips, each injection tip may be independently controllable.

The endotracheal tube 2200, fluid lines 2212, 2216, injection tip(s) 2210, and/or other features or components of the endotracheal tube 2200, internal or external, may include an anti-microbial coating and/or an anti-microbial material (e.g., silver alloy) to minimize build-up or clogs of the injection tip (e.g., from humidity, fluids from the patient, etc.). The coating and/or material may be hydrophobic and/or oleophobic.

Although above descriptions of nebulizer systems describe injection of dispersed fluid droplets in or along the breathing circuit, at the wye component, at an endotracheal tube coupling component, and at an endotracheal tube, injection of aerosolized fluid into gases for delivery to a patient at any component are appreciated. For example, dispersed fluid droplets may be integrated into laryngeal mask airway (LMA) systems, a nasal cannula, a mask, a breathing circuit filter (e.g., on the patient side of the breathing circuit), a catheter mount, tracheostomy systems, bronchial catheter systems, any airway circuit connector, or any other airway access device. Additionally, a lumen and/or nozzle similar to those described herein may be threaded through a wall of any other patient interface or other component along the breathing circuit.

A person of skill in the art will understand that the technology described in the context of a medical ventilator for human patients could be adapted for use with other systems such as ventilators for non-human patients or general gas transport systems. Additionally, a person of ordinary skill in the art will understand that a fluid delivery system and/or a humidification system may be implemented in a variety of breathing circuit setups with a variety of patient interfaces.

Those skilled in the art will recognize that the methods and systems of the present disclosure may be implemented in many manners and as such are not to be limited by the foregoing aspects and examples. In other words, functional elements being performed by a single or multiple components, in various combinations of hardware and software or firmware, and individual functions, can be distributed among software applications at either the client or server level or both. In this regard, any number of the features of the different aspects described herein may be combined into single or multiple aspects, and alternate aspects having fewer than or more than all of the features herein described are possible.

Functionality may also be, in whole or in part, distributed among multiple components, in manners now known or to become known. Thus, a myriad of software/hardware/firmware combinations are possible in achieving the functions, features, interfaces and preferences described herein. Moreover, the scope of the present disclosure covers conventionally known manners for carrying out the described features and functions and interfaces, and those variations and modifications that may be made to the hardware or software firmware components described herein as would be understood by those skilled in the art now and hereafter. In addition, some aspects of the present disclosure are described above with reference to block diagrams and/or operational illustrations of systems and methods according to aspects of this disclosure. The functions, operations, and/or acts noted in the blocks may occur out of the order that is shown in any respective flowchart. For example, two blocks shown in succession may in fact be executed or performed substantially concurrently or in reverse order, depending on the functionality and implementation involved.

Further, as used herein and in the claims, the phrase "at least one of element A, element B, or element C" is intended to convey any of: element A, element B, element C, elements A and B, elements A and C, elements B and C, and elements A, B, and C. In addition, one having skill in the art will understand the degree to which terms such as "about" or "substantially" convey in light of the measurements techniques utilized herein. To the extent such terms may not be clearly defined or understood by one having skill in the art, the term "about" shall mean plus or minus ten percent.

Numerous other changes may be made which will readily suggest themselves to those skilled in the art and which are encompassed in the disclosure. While various aspects have been described for purposes of this disclosure, various changes and modifications may be made which are well within the scope of the disclosure. Numerous other changes may be made which will readily suggest themselves to those skilled in the art and which are encompassed in the disclosure.

The scope of the present invention is defined by the following appended claims.

## Claims

1. A fluid delivery system (124) for delivery of aerosolized liquid to a ventilated patient (150) comprising:
an endotracheal tube (182) configured to deliver breathing gases to a patient (150);
a multi-port connector (300) coupled to the endotracheal tube (182), the multi-port connector (300) including:
a primary channel (306) directing a flow of the breathing gases into the endotracheal tube (182); and
a secondary channel (312) having a first end (310) and a second end (320),
wherein the secondary channel (312) is fluidly coupled to the primary channel (306) at the second end (320) at an angle that is 90 degrees or less from the flow of the breathing gases;
a pressure applicator (406); and **characterised by**:
an injection tip (404) configured to engage with the first end (310) of the secondary channel (312) and the pressure applicator (406), wherein the injection tip (404) includes a set of orifices configured to aerosolize a liquid when the liquid is applied at a pressure by the pressure applicator (406).

2. The fluid delivery system (124) of claim 1, wherein the pressure applicator (406) is one of: a syringe or a pump.

3. A dual nozzle ventilation system (900) comprising:
a humidification system (928) including:
a first pump (934) configured to pressurize water from a water reservoir (930);
a first nozzle (944) configured to atomize the water, wherein the first nozzle (944) is positioned in a heated portion of a ventilation tubing configured to deliver breathing gases to a ventilated patient (150) such that atomized water interacts with the heated portion of the ventilation tubing to humidify the breathing gases;
a first valve (936) to control the flow of pressurized water from the first pump into the first nozzle; and
a first controller (938) configured to control the first valve (936) based on a flowrate of the breathing gases; and
the fluid delivery system (124) of claim 1, wherein the pressure applicator (406) comprises:
a second pump (910) configured to pressurize a liquid from a fluid reservoir (908); a second nozzle (904) positioned downstream in the line from the first nozzle (944) of the humidification system (928), wherein the second nozzle (904) is configured to aerosolize the liquid through the set of orifices;
a second valve (912) to control the flow of pressurized liquid from the second pump (910) into the second nozzle (904); and
a second controller (914) configured to control the second valve (912) based on the flowrate of the breathing gases.

4. The dual nozzle ventilation system (900) of claim 3, wherein the ventilation tubing includes a wye (922) and wherein the first nozzle (944) and the second nozzle (904) are positioned downstream of the wye (922).

5. The dual nozzle ventilation system (900) of claim 3, wherein the ventilation tubing includes a wye (922) and wherein the first nozzle (944) is positioned upstream of the wye (922) and the second nozzle (904) is positioned downstream of the wye (922).

6. The dual nozzle ventilation system (900) of claim 5, wherein the second nozzle (904) is coupled to the multi-port connector (300) coupled to the endotracheal tube (182).

7. The dual nozzle ventilation system (900) of claim 6, wherein
the humidified breathing gases are configured to flow into a primary port (302) of the multi-port connector (300);
wherein the primary channel (306) extends through the primary port (302); and the second nozzle (904) is coupled to a secondary port (308) of the multi-port connector (300);
wherein the secondary channel (312) extends through the secondary port (308).

## Patentansprüche

1. Fluidabgabesystem (124) für Abgabe aerosolisierter Flüssigkeit an einen beatmeten Patienten (150), umfassend:
einen Endotrachealtubus (182), der konfiguriert ist, um an einen Patienten (150) Atemgase abzugeben;
einen Mehrport-Verbinder (300), der mit dem Endotrachealtubus (182) gekoppelt ist, der Mehrport-Verbinder (300) einschließlich:
eines Primärkanals (306), der einen Strom der Atemgase in den Endotrachealtubus (182) lenkt; und
eines Sekundärkanals (312), der ein erstes Ende (310) und ein zweites Ende (320) aufweist, wobei der Sekundärkanal (312) an dem zweiten Ende (320) in einem Winkel von 90 Grad oder weniger von der Strömung der Atemgase mit dem Primärkanal (306) fluidisch gekoppelt ist;
einen Druckapplikator (406); und **gekennzeichnet durch:**
eine Injektionsspitze (404), die konfiguriert ist, um das erste Ende (310) des Sekundärkanals (312) und den Druckapplikator (406) in Eingriff zu nehmen, wobei die Injektionsspitze (404) einen Satz von Öffnungen umfasst, die konfiguriert sind, um eine Flüssigkeit zu aerosolisieren, wenn die Flüssigkeit durch den Druckapplikator (406) mit einem Druck aufgebracht wird.

2. Fluidabgabesystem (124) nach Anspruch 1, wobei der Druckapplikator (406) eine ist von: einer Spritze oder einer Pumpe.

3. Doppeldüsen-Beatmungssystem (900), umfassend:
ein Befeuchtungssystem (928), einschließlich:
einer ersten Pumpe (934), die konfiguriert ist, um Wasser aus einem Wasserbehälter (930) unter Druck zu setzen;
einer erste Düse (944), die konfiguriert ist, um das Wasser zu zerstäuben, wobei die erste Düse (944) in einem erwärmten Abschnitt einer Beatmungsleitung positioniert ist, die konfiguriert ist, um Atemgase an einen beatmeten Patienten (150) abzugeben, derart, dass zerstäubtes Wasser mit dem erwärmten Abschnitt der Beatmungsleitung interagiert, um die Atemgase zu befeuchten;
eines ersten Ventils (936), um den Strom unter Druck gesetzten Wassers von der ersten Pumpe in die erste Düse zu steuern; und
einer ersten Steuervorrichtung (938), die konfiguriert ist, um das erste Ventil (936) basierend auf einer Durchflussgeschwindigkeit der Atemgase zu steuern; und
das Fluidabgabesystem (124) nach Anspruch 1, wobei der Druckapplikator (406) umfasst:
eine zweite Pumpe (910), die konfiguriert ist, um eine Flüssigkeit aus einem Fluidbehälter (908) unter Druck zu setzen; eine zweite Düse (904), die in der Leitung von der ersten Düse (944) des Befeuchtungssystems (928) stromabwärts positioniert ist, wobei die zweite Düse (904) konfiguriert ist, um die Flüssigkeit durch den Satz von Öffnungen zu aerosolisieren;
ein zweites Ventil (912), um den Strom unter Druck gesetzter Flüssigkeit von der zweiten Pumpe (910) in die zweite Düse (904) zu steuern; und
eine zweite Steuervorrichtung (914), die konfiguriert ist, um das zweite Ventil (912) basierend auf der Durchflussgeschwindigkeit der Atemgase zu steuern.

4. Doppeldüsen-Beatmungssystem (900) nach Anspruch 3, wobei die Beatmungsleitung ein Y-Rohr (922) einschließt, und wobei die erste Düse (944) und die zweite Düse (904) stromabwärts des Y-Rohrs (922) positioniert sind.

5. Doppeldüsen-Beatmungssystem (900) nach Anspruch 3, wobei die Beatmungsleitung ein Y-Rohr (922) einschließt, und wobei die erste Düse (944) stromaufwärts des Y-Rohrs (922) positioniert ist und die zweite Düse (904) stromabwärts des Y-Rohrs (922) positioniert ist.

6. Doppeldüsen-Beatmungssystem (900) nach Anspruch 5, wobei die zweite Düse (904) mit dem Mehrport-Verbinder (300) gekoppelt ist, der mit dem Endotrachealtubus (182) gekoppelt ist.

7. Doppeldüsen-Beatmungssystem (900) nach Anspruch 6, wobei:
die befeuchteten Atemgase konfiguriert sind, um in einen Primärport (302) des Mehrport-Verbinders (300) zu strömen;
wobei sich der Primärkanal (306) durch den Primärport (302) erstreckt; und
die zweite Düse (904) mit einem Sekundärport (308) des Mehrport-Verbinders (300) gekoppelt ist;
wobei sich der Sekundärkanal (312) durch den Sekundärport (308) erstreckt.

## Revendications

1. Système d'administration de fluide (124) destiné à l'administration d'un liquide aérosolisé à un patient ventilé (150) comprenant :
un tube endotrachéal (182) conçu pour administrer des gaz respiratoires à un patient (150) ;
un connecteur multiport (300) accouplé au tube endotrachéal (182), le connecteur multiport (300) comportant :
un canal primaire (306) dirigeant un flux des gaz respiratoires dans le tube endotrachéal (182) ; et
un canal secondaire (312) ayant une première extrémité (310) et une seconde extrémité (320), dans lequel le canal secondaire (312) est accouplé fluidiquement au canal primaire (306) au niveau de la seconde extrémité (320) à un angle de 90 degrés ou moins par rapport à l'écoulement des gaz respiratoires ;
un applicateur de pression (406) ; et **caractérisé par :**
un embout d'injection (404) conçu pour venir en prise avec la première extrémité (310) du canal secondaire (312) et l'applicateur de pression (406), dans lequel l'embout d'injection (404) comporte un ensemble d'orifices conçus pour aérosoliser un liquide lorsque le liquide est appliqué à une pression par l'applicateur de pression (406).

2. Système d'administration de fluide (124) selon la revendication 1, dans lequel l'applicateur de pression (406) est l'un parmi : une seringue ou une pompe.

3. Système de ventilation à double buse (900) comprenant :
un système d'humidification (928) comportant :
une première pompe (934) conçue pour pressuriser l'eau d'un réservoir d'eau (930) ;
une première buse (944) conçue pour atomiser l'eau, dans laquelle la première buse (944) est positionnée dans une partie chauffée d'un tuyau de ventilation configuré pour administrer des gaz respiratoires à un patient ventilé (150) de telle sorte que l'eau atomisée interagisse avec la partie chauffée du tuyau de ventilation pour humidifier les gaz respiratoires ;
une première vanne (936) pour commander l'écoulement de l'eau pressurisée de la première pompe dans la première buse ; et
un premier contrôleur (938) configuré pour commander la première vanne (936) en fonction d'un débit des gaz respiratoires ; et
système d'administration de fluide (124) selon la revendication 1, dans lequel l'applicateur de pression (406) comprend :
une seconde pompe (910) conçue pour pressuriser un liquide provenant d'un réservoir de fluide (908) ; une seconde buse (904) placée en aval en ligne de la première buse (944) du système d'humidification (928), dans lequel la seconde buse (904) est conçue pour aérosoliser le liquide à travers l'ensemble des orifices ;
une seconde valve (912) pour commander le flux de liquide pressurisé de la seconde pompe (910) dans la seconde buse (904) ; et
un second contrôleur (914) configuré pour commander la seconde valve (912) en fonction du débit des gaz respiratoires.

4. Système de ventilation à double buse (900) selon la revendication 3, dans lequel le tuyau de ventilation comporte un coude (922) et dans lequel la première buse (944) et la seconde buse (904) sont positionnées en aval du coude (922).

5. Système de ventilation à double buse (900) selon la revendication 3, dans lequel le tuyau de ventilation comporte un coude (922) et dans lequel la première buse (944) est positionnée en amont du coude (922) et la seconde buse (904) est positionnée en aval du coude (922).

6. Système de ventilation à double buse (900) selon la revendication 5, dans lequel la seconde buse (904) est accouplée au connecteur multiport (300) accouplé au tube endotrachéal (182).

7. Système de ventilation à double buse (900) selon la revendication 6, dans lequel :
les gaz respiratoires humidifiés sont conçus pour s'écouler dans un orifice primaire (302) du connecteur multiport (300) ;
dans lequel le canal primaire (306) s'étend à travers l'orifice primaire (302) ; et
la seconde buse (904) est accouplée à un port secondaire (308) du connecteur multiport (300) ;
dans lequel le canal secondaire (312) s'étend à travers l'orifice secondaire (308).
